# EUROPEAN PATENT APPLICATION

(11) **EP 4 250 302 A2**
(43) Date of publication of application: **27.09.2023**
(21) Application number: 23169895.2
(22) Date of filing: 23.02.2011
(51) Int. Cl.: G16C 10/00

(54) **METHODS AND SYSTEMS FOR PROVIDING THERAPEUTIC GUIDELINES TO A PERSON HAVING DIABETES**

(30) Priority: 23.02.2010 US 71043010
(62) Divisional of application: 11714655.5
(71) Applicant: Roche Diabetes Care GmbH, 68305 Mannheim (DE); F. Hoffmann-La Roche AG, 4070 Basel (CH)
(72) Inventor: Wagner, Robin, Indianapolis, 46220 (US); Weinert, Stefan, Pendleton, 46064 (US); Laan, Remmert, Zionsville, 46077 (US); Waechter, Kurt, Indianapolis, 46205 (US); Rees, Christen, Indianapolis, 46256 (US); Atkinson, Craig, Frenchs Forest, 2086 (AU); Soni, Abhishek, Indianapolis, 46280 (US); Duke, David, Fishers, 46037 (US)
(74) Representative: Bittner, Thomas L.

(57) **Abstract**

A method is disclosed that comprises detecting a pattern of an abnormality in blood glucose data collected from an individual with a computing device comprising a processor; generating a change in therapy recommendation for the individual with the computing device based on said detecting the pattern; and outputting a customized testing protocol customized to detect whether the change in therapy successfully addressed the abnormality with the computing device. Further, a system is disclosed.

## Description

The present invention generally relates to methods and systems for providing therapeutic guidelines to a person having diabetes.

### BACKGROUND

As background, people may suffer from either Type I or Type II diabetes in which the glucose level in the blood is not properly regulated by the body. Many of these people monitor their own blood glucose levels throughout the day by using blood glucose meters. For example, a person may measure his or her blood glucose level before and after each meal.

Furthermore, a health care provider may recommend a therapeutic regimen for the person having diabetes. The regimen may provide advice on eating, exercising, and so forth, and may facilitate keeping the person's blood glucose level within a desired range. Since many factors may affect the blood glucose level of a person, it may be helpful to periodically review the history of the person's blood glucose level and determine whether and how closely the blood glucose level stays within the desired range.

Accordingly, embodiments of the present disclosure provide methods and systems for determining whether a person's blood glucose level falls within the desired range and, if not, for providing therapeutic guidelines to the person, based on the measured blood glucose levels.

### SUMMARY

In one embodiment, a method for providing therapeutic guidelines to a person having diabetes comprises: measuring a blood glucose (bG) level of the person for two or more days, wherein at least one bG measurement is taken per day, and the at least one daily bG measurement corresponds to one or more daily events for the person; recording the measured bG levels in a computing device; determining, by the computing device, whether the recorded bG levels are below, within, or above one or more predetermined bG ranges; and automatically providing, by the computing device, therapeutic guidelines to the person, based on whether the recorded bG levels are below, within, or above the one or more predetermined bG ranges.

In another embodiment, a computer-readable medium having computer- executable instructions for performing a method for providing therapeutic guidelines to a person having diabetes is disclosed. The method comprises: receiving measured blood glucose (bG) levels of the person into a computing device, wherein the measured bG levels of the person are taken for two or more days such that at least one bG measurement is taken per day, and the at least one daily bG measurement corresponds to one or more daily events for the person; recording the measured bG levels in the computing device; determining, by the computing device, whether the recorded bG levels are below, within, or above one or more predetermined bG ranges; and automatically providing, by the computing device, therapeutic guidelines to the person, based on whether the recorded bG levels are below, within, or above the one or more predetermined bG ranges.

In still another embodiment, a blood glucose meter for providing therapeutic guidelines to a person having diabetes comprises a processor, a memory, a display readable by the person, and a measuring element, wherein: the measuring element is configured to measure the blood glucose (bG) level of the person for two or more days, wherein at least one bG measurement is taken per day, and the at least one daily bG measurement corresponds to one or more daily events for the person; the processor is in electrical communication with the measuring element such that the processor is configured to read the bG level of the person measured by the measuring element; the processor is in electrical communication with the memory such that the processor is configured to record the measured bG levels in the memory; the memory comprises one or more predetermined bG ranges, such that the processor is configured to read the one or more predetermined bG ranges and the recorded bG levels and determine whether the recorded bG levels are below, within, or above the one or more predetermined bG ranges; and the processor is in electrical communication with the display such that the processor is configured to transmit therapeutic guidelines to the display, wherein the therapeutic guidelines are based on whether the recorded bG levels are below, within, or above the one or more predetermined bG ranges.

A method for providing therapeutic guidelines to a person having diabetes may comprise measuring a blood glucose (bG) level of the person for two or more days, wherein at least one bG measurement is taken per day, and the at least one daily bG measurement corresponds to one or more daily events for the person; recording the measured bG levels in a computing device; determining, by the computing device, whether the recorded bG levels are below, within, or above one or more predetermined bG ranges; and automatically providing, by the computing device, therapeutic guidelines to the person, based on whether the recorded bG levels are below, within, or above the one or more predetermined bG ranges.

The measuring of the bG level of the person may take place for three or more consecutive days.

The one or more daily events for the person may comprise eating breakfast, eating lunch, eating dinner, and going to sleep, such that at least seven bG measurements are taken per day: one before and after the person eats breakfast, lunch, and dinner, and one before the person goes to sleep. Herein, the bG measurements taken after the person eats breakfast, lunch, and dinner may be taken approximately two hours after the person eats breakfast, lunch, and dinner. The one or more predetermined bG ranges may comprise: a first predetermined bG range of approximately 81 milligrams of glucose per deciliter of blood (mg/dl) to approximately 110 mg/dl for bG measurements taken before the person eats breakfast, lunch, and dinner; and a second predetermined bG range of approximately 81 mg/dl to approximately 140 mg/dl for measurements taken after the person eats breakfast, lunch, and dinner and before the person goes to sleep.

The method may further comprise determining whether the recorded bG levels are below or above the one or more predetermined bG ranges by at least a bG excursion amount, wherein the determining is performed by the computing device, and wherein automatically providing therapeutic guidelines to the person is further based on whether the recorded bG levels are below or above the one or more predetermined bG ranges by at least the bG excursion amount. Herein, the bG excursion amount may be approximately 50 milligrams of glucose per deciliter of blood.

A computer-readable medium may be provided having computer-executable instructions for performing a method for providing therapeutic guidelines to a person having diabetes, the method comprising: receiving measured blood glucose (bG) levels of the person into a computing device, wherein the measured bG levels of the person are taken for two or more days such that at least one bG measurement is taken per day, and the at least one daily bG measurement corresponds to one or more daily events for the person; recording the measured bG levels in the computing device; determining, by the computing device, whether the recorded bG levels are below, within, or above one or more predetermined bG ranges; and automatically providing, by the computing device, therapeutic guidelines to the person, based on whether the recorded bG levels are below, within, or above the one or more predetermined bG ranges.

The bG levels of the person may be measured for three or more consecutive days.

The one or more daily events for the person may comprise eating breakfast, eating lunch, eating dinner, and going to sleep, such that at least seven bG measurements are taken per day: one before and after the person eats breakfast, lunch, and dinner, and one before the person goes to sleep. Herein, the bG levels of the person measured after the person eats breakfast, lunch, and dinner may be measured approximately two hours after the person eats breakfast, lunch, and dinner. The one or more predetermined bG ranges may comprise: a first predetermined bG range of approximately 81 milligrams of glucose per deciliter of blood (mg/dl) to approximately 110 mg/dl for measured bG levels taken before the person eats breakfast, lunch, and dinner; and a second predetermined bG range of approximately 81 mg/dl to approximately 140 mg/dl for measured bG levels taken after the person eats breakfast, lunch, and dinner and before the person goes to sleep.

The computer-readable medium may further comprise determining whether the recorded bG levels are below or above the one or more predetermined bG ranges by at least a bG excursion amount, wherein the determining is performed by the computing device, and wherein automatically providing therapeutic guidelines to the person is further based on whether the recorded bG levels are below or above the one or more predetermined bG ranges by at least the bG excursion amount. Herein, the bG excursion amount may be approximately 50 milligrams of glucose per deciliter of blood.

The computer-readable medium may comprise a compact disc (CD), a USB thumb drive, an optical drive, or a magnetic drive.

A blood glucose meter for providing therapeutic guidelines to a person having diabetes may comprise a processor, a memory, a display readable by the person, and a measuring element, wherein: the measuring element is configured to measure the blood glucose (bG) level of the person for two or more days, wherein at least one bG measurement is taken per day, and the at least one daily bG measurement corresponds to one or more daily events for the person; the processor is in electrical communication with the measuring element such that the processor is configured to read the bG level of the person measured by the measuring element; the processor is in electrical communication with the memory such that the processor is configured to record the measured bG levels in the memory; the memory comprises one or more predetermined bG ranges, such that the processor is configured to read the one or more predetermined bG ranges and the recorded bG levels and determine whether the recorded bG levels are below, within, or above the one or more predetermined bG ranges; and the processor is in electrical communication with the display such that the processor is configured to transmit the therapeutic guidelines to the display, wherein the therapeutic guidelines are based on whether the recorded bG levels are below, within, or above the one or more predetermined bG ranges.

Measuring the bG levels of the person may take place for three or more consecutive days.

The one or more daily events for the person may comprise eating breakfast, eating lunch, eating dinner, and going to sleep, such that at least seven bG measurements are taken per day: one before and after the person eats breakfast, lunch, and dinner, and one before the person goes to sleep. Herein, the bG measurements taken after the person eats breakfast, lunch, and dinner may be taken approximately two hours after the person eats breakfast, lunch, and dinner. The one or more predetermined bG ranges may comprise: a first predetermined bG range of approximately 81 milligrams of glucose per deciliter of blood (mg/dl) to approximately 110 mg/dl for bG measurements taken before the person eats breakfast, lunch, and dinner; and a second predetermined bG range of approximately 81 mg/dl to approximately 140 mg/dl for measurements taken after the person eats breakfast, lunch, and dinner and before the person goes to sleep.

The blood glucose meter may further comprise determining whether the recorded bG levels are below or above the one or more predetermined bG ranges by at least a bG excursion amount, and providing the therapeutic guidelines to the person is further based on whether the recorded bG levels are below or above the one or more predetermined bG ranges by at least the bG excursion amount. Herein, the bG excursion amount may be approximately 50 milligrams of glucose per deciliter of blood.

A method may comprise: detecting a pattern of an abnormality in blood glucose data collected from an individual with a computing device; generating a change in therapy recommendation for the individual with the computing device based on said detecting the pattern; and outputting a customized testing protocol customized to detect whether the change in therapy successfully addressed the abnormality with the computing device.

The method may further comprise receiving the blood glucose data with the computing device from a standardized structured testing data collection form before said detecting the pattern. Said receiving the blood glucose data may include downloading the blood glucose data from a blood glucose meter. Said receiving the blood glucose data may include scanning a paper version of the structured testing data collection form.

The method may comprise: confirming the change in therapy successfully addressed the abnormality by analyzing data from the customized testing protocol with the computing device; instructing the individual to collect a second set of blood glucose data with the standardized structured testing data collection form with the computing device; and analyzing the second set of blood glucose data for a second abnormality pattern with the computing device.

The method may comprise: receiving background information about the individual with the computing device; and wherein said generating the change in therapy recommendation includes selecting the change in therapy recommendation based at least in part on the background information. Herein, the background information may include demographic information. The background information may include comorbidity information. The background information may include medication information. The background information may include diabetes duration.

The background information may include social media preferences for the individual; and said generating the change in therapy recommendation may include providing a social media advice component based at least on the social media preferences of the individual.

The method may comprise detecting the abnormality in the blood glucose data with the computing device before said detecting the pattern.

The method may comprise: asking one or more assessment questions with the computing device at least based on the pattern of the abnormality; and wherein said generating the change in therapy recommendation is at least based on answers to the assessment questions.

The change in therapy may include a change in medication recommendation. Herein, the method may further comprise providing the change in medication recommendation to a physician.

The change in therapy may include a change in lifestyle. Herein the change in lifestyle may include a change in exercise. The change in lifestyle may include a change in diet.

The method may further comprise: determining the abnormality is severe with the computing device; and notifying a health care provider that the abnormality is severe.

The abnormality may include hypoglycemia. Herein, the pattern may include repeated waking hypoglycemia.

The abnormality may include hyperglycemia. Herein, the pattern may include repeated postprandial hyperglycemia. The pattern may include repeated preprandial hyperglycemia.

Said outputting the customized testing protocol may include printing a customized structured testing form with a printer. Said outputting the customized testing protocol may include displaying a customized structured testing form on a computer display. Said outputting the customized testing protocol may include providing advice related to the therapy recommendation.

The computing device may include a personal computer. The computing device may include a blood glucose meter. The computing device may include a web hosted computer system.

A method may comprise: detecting a pattern for a blood glucose abnormality with a computing device base on blood glucose data and contextual data collected from an individual; and generating a change in therapy recommendation for the individual automatically with the computing device based on said detecting the pattern.

The method may further comprise outputting a customized testing protocol customized to detect whether the change in therapy successfully addressed the blood glucose abnormality with the computing device.

The contextual data may include data about the individual surrounding collection of the blood glucose data. The contextual data may include dietary information for the individual. The contextual data may include activity information for the individual.

Said detecting the pattern may include considering more than one type of the contextual data with the computing device.

Said generating the change in therapy recommendation the computing device may take into account more than one parameter.

During said generating the change, in therapy recommendation, the computing device may take into account relationships between parameters.

A method may comprise: detecting a pattern for a blood glucose abnormality with a computing device base on blood glucose data and contextual data collected from an individual; and generating a threat alert for the individual automatically with the computing device based on said detecting the pattern. The method may further comprise: wherein the blood glucose abnormality includes hypoglycemia; and wherein said generating the threat alert includes displaying the threat alert on a glucose meter.

The method may further comprise: collecting the blood glucose data and the contextual data within a window that spans before and after when the blood glucose abnormality occurs; and wherein said collecting includes collecting the blood glucose data and the contextual data at predefined intervals within the window. Herein, the window may be three hours and the predefined interval may be 15 to 20 minutes.

The method may comprise: wherein said detecting the pattern for the blood glucose abnormality is performed with a continuous blood glucose monitoring device; and retesting the individual with a discrete testing glucose meter to reconfirm the abnormality.

In the method, all or part of the acts may be performed by a personal computer. All or part of the acts may be performed by a blood glucose meter. All or part of the acts may be performed by a continuous blood glucose monitoring device.

A system configured to perform the combination of acts recited above may be provided.

A system may comprise: means for detecting a pattern of an abnormality in blood glucose data collected from an individual; means for generating a change in therapy recommendation for the individual based on the pattern; and means for outputting a customized testing protocol customized to detect whether the change in therapy successfully addressed the abnormality. Herein, the means for detecting the pattern may include a computing device.

### BRIEF DESCRIPTION OF THE DRAWINGS

The embodiments set forth in the drawings are illustrative and exemplary in nature and not intended to limit the inventions defined by the claims. The following detailed description of the illustrative embodiments can be understood when read in conjunction with the following drawings, where like structure is indicated with like reference numerals and in which:
- FIG. 1: depicts a blood glucose meter according to one or more embodiments shown and described herein;
- FIG. 2: depicts a personal computer according to one or more embodiments shown and described herein;
- FIG. 3: depicts a flow diagram of one method for providing therapeutic guidelines according to one or more embodiments shown and described herein;
- FIG. 4: depicts a flow diagram of one method for providing therapeutic guidelines according to one or more embodiments shown and described herein;
- FIGS. 5A-B: depict a flow diagram of one method for determining therapeutic guidelines according to one or more embodiments shown and described herein; and
- FIG. 6: depicts therapeutic guidelines according to one or more embodiments shown and described herein.
- FIGS. 7A and 7B: depict a flow diagram illustrating one method for developing a customized therapy regimen and a customized structured test.
- FIG. 8: depicts a standardized structured testing form used in conjunction with the method described with reference to FIGS. 7 A and 7B.
- FIG. 9: depicts a customized structured testing form used to determine the effectiveness of intermediate-acting insulin.
- FIG. 10: depicts a customized structured testing form used to determine the effectiveness of a change in exercise.
- FIG. 11: depicts a customized structured testing form used to determine the effectiveness of a change in diet.
- FIG. 12: is a flow diagram that illustrates a technique for developing a threat alert for hypoglycemic events.
- FIG. 13: is a flow diagram that illustrates a technique for alerting a user of a potential hypoglycemic event.

### DETAILED DESCRIPTION

The embodiments described herein generally relate to methods and systems for providing therapeutic guidelines to people having diabetes.

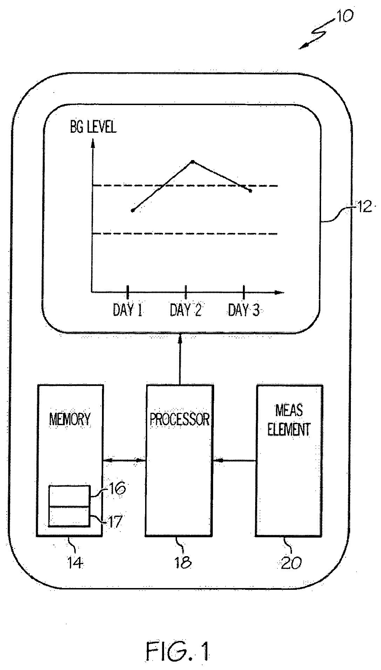

FIG. 1 depicts a blood glucose (bG) meter 10 according to one embodiment of the present disclosure. The bG meter 10 may comprise a display 12, a memory 14, a processor 18, and a measuring element 20. The measuring element 20 may be configured to measure the bG level of a person such as, for example, by using a blood sample from the person. The measuring element 20 may be in electrical communication with the processor 18 such that the processor is configured to read the bG measurement from the measuring element 20. The memory 14 may be in electrical communication with the processor 18 such that the processor 18 may record (or store) the bG measurement in the memory 14. The processor 18 may be configured to read a plurality of bG measurements per day from the measuring element 20 and may be configured to record each of these bG measurements in the memory 14. Furthermore, the processor 18 may be configured to record bG measurements from two or more days in the memory 14. For example, one month's worth of bG measurements may be recorded in the memory 14. The bG meter 10 may be configured such that it may transmit some or all of the stored bG measurements to another device, either via a wired or wireless connection (not shown).

The memory 14 may also comprise one or more predetermined bG ranges 16 for the person. As an example, a first predetermined bG range 16 may be approximately 81 to approximately 140 mg/dl (milligrams of glucose per deciliter of blood), while a second predetermined bG range may be approximately 81 to approximately 110 mg/dl. Other ranges may be used as well and may depend on the characteristics of the person. Blood glucose levels which fall below the predetermined bG range (i.e., below 81 mg/dl in the above examples) may be considered "hypoglycemic." Similarly, blood glucose levels which fall above the predetermined bG range (i.e., above 140 mg/dl for the first range or 1 10 mg/dl for the second range in the above examples) may be considered "hyperglycemic." Consequently, blood glucose levels which fall within these predetermined bG ranges may be considered "normal." As disclosed herein, one or more predetermined bG ranges may be used, such that a first predetermined bG range may be used for some of the measured bG results, while a second predetermined bG range may be used for other measured bG results. Any number of predetermined bG ranges may be used.

The memory 14 may further comprise a blood glucose (bG) excursion amount 17. Blood glucose levels which are below the predetermined bG range 16 by at least the bG excursion amount may be considered "severe hypoglycemic." Similarly, blood glucose levels which are above the predetermined bG range 16 by at least the bG excursion amount 17 may be considered "severe hyperglycemic." As an example, the predetermined bG range 16 may be 81 to 140 mg/dl, and bG excursion amount may be 50 mg/dl. In this example, a bG level of 141 to 189 mg/dl may be considered hyperglycemic; and a bG level of 190 mg/dl and above may be considered severe hyperglycemic. Continuing with this example, a bG level of 31 to 80 mg/dl may be considered hypoglycemic; and a bG level of 30 mg/dl and below may be considered severe hypoglycemic. Whether the person's bG level falls below, within, or above a predetermined amount may be subsequently used by the processor 18 to provide therapeutic guidelines to the person.

The bG meter 10 may further comprise a display 12, which may be readable by the operator. The display 12 may be in electrical communication with the processor 18 such that the processor is configured to send information to the display 12. As an example, the processor 18 may send either graphical or textual information to the display 12 which may provide therapeutic guidelines to the operator. Graphical information may include X-Y graphs of the person's bG level history or other suitable information. Textual information may include text messages, such as "Your bG level is 117 mg/dl." Both graphical and textual information may be display simultaneously, if desired. The display 12 may be a liquid crystal display (LCD) or other suitable display.

The bG meter 10 may be configured to measure the bG level of the person for two or more days. At least one bG measurement may be taken per day, and each bG measurement may correspond to at least one daily event for the person.

Generally, the daily events may take place at approximately the same time each day. Daily events may include, but are not limited to, eating breakfast, eating lunch, eating dinner, and going to sleep. Other daily events may be used as well, including daily events which may affect the bG level of the person such as, but not limited to, exercising and taking medication. Regarding the three daily meals, breakfast generally may be eaten in the morning, lunch may be eaten around noon, and dinner may be eaten in the late afternoon or evening, although the meals may be eaten at other times as well, depending on the person's sleep schedule. As an example, a person working third shift (e.g., working approximately midnight to 8:00 am) may eat "dinner" at 9:00 am, may sleep from noon to 8:00 pm, may eat "breakfast" at 8:30 pm, and may eat "lunch" at 1 :00 am. Other such sleep or eating schedules are contemplated as well.

The bG meter 10 may be configured to measure the person's bG level for three consecutive days, for example. For each day, the bG meter 10 may be configured to measure the person's bG level before and after each of the three daily meals (i.e., breakfast, lunch, and dinner) as well as before the person goes to sleep. In this fashion, the bG meter may be configured to take at least seven bG measurements per day. In addition, the person's bG level may be taken approximately two hours after ingesting a meal. This may provide a more accurate bG level measurement for the person.

After the bG measurements for the two or more days have been recorded in the memory 14, the processor 18 may be configured to read the bG measurements and the one or more predetermined bG ranges 16 from the memory 14. The processor 18 may then determine whether each bG measurement falls below, within, or above one of the one or more predetermined bG ranges. Based on these determinations, the processor 18 may be configured to transmit therapeutic guidelines to the display 12.

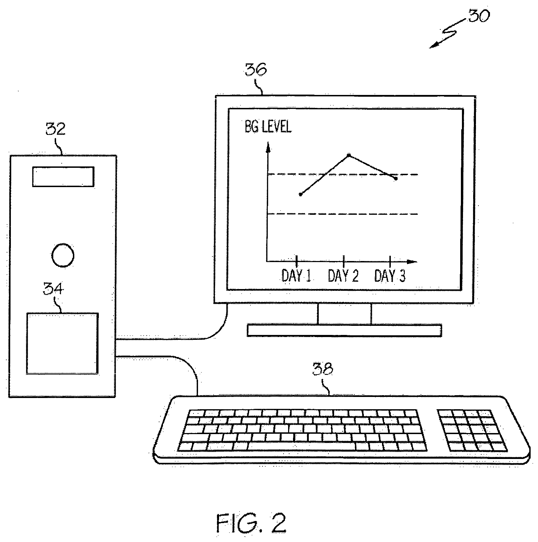

FIG. 2 illustrates another embodiment of the present disclosure in which a computing device 30 may comprise a processor 32, memory 34, a display 36, and an input device 38. The processor 32, memory 34, and display 36 may function in the same manner as the same-named elements shown in FIG. 1 and described herein. The input device 38 may comprise a keyboard, such as a "hard keyboard," which has physical, dedicated buttons the person may press. Alternatively, input device 38 may comprise a touch screen (not shown), which permits the person to enter information by pressing certain locations on the display 36. Other types of input devices may be used as well, as is known in the art.

The computing device 30, although depicted as a desktop personal computer in FIG. 2, may also be a laptop computer, a cellular phone, a smart phone, a personal digital assistant, or any suitable device. The computing device 30 may be configured to allow the bG measurements to be manually entered into the computing device 30 via the input device 38. As an example, the person may type the bG measurements into the computing device 30 through a keyboard. Alternatively, the bG measurements may be transmitted to the computing device 30 through a wired or a wireless interface. For example, the computing device 30 may wirelessly receive bG measurements from a bG meter via a Bluetooth interface. In this fashion, the computing device 30 may automatically receive the bG measurements. Once the computing device 30 has received the bG measurements, it may record the measurements, determine whether the measurements are below, within, or above the predetermined bG range, and provide therapeutic guidelines to the person, based on whether the recorded bG levels are below, within, or above the predetermined bG range.

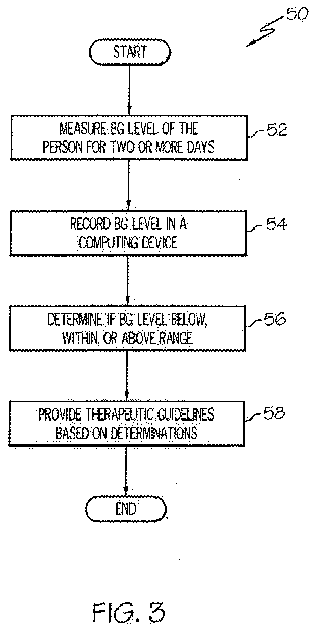

FIG. 3 depicts a flow diagram 50 of a method for providing therapeutic guidelines to a person having diabetes. This method may be performed on a bG meter, such as the one shown in FIG. 1 or any other suitable device. Act 52 of the method may measure a blood glucose (bG) level of the person for two or more days, wherein at least one bG measurement is taken per day, and the at least one daily bG measurement corresponds to at least one daily event for the person. Act 54 of the method may record the measured bG levels in a computing device. Act 56 of the method may determine whether the recorded bG levels are below, within, or above one or more predetermined bG ranges. And act 58 of the method may provide therapeutic guidelines to the person, based on whether the recorded bG levels are below, within, or above the one or more predetermined bG ranges. The acts of the method may be performed in any suitable order.

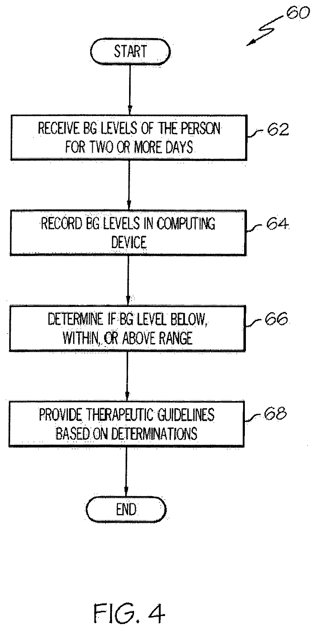

FIG. 4 depicts another flow diagram 60 of a method for providing therapeutic guidelines to a person having diabetes. This method may be stored on a computer-readable medium having computer-executable instructions for performing the method. A computer-readable medium may include, but is not limited to, a compact disc (CD), a USB thumb drive, an optical drive, or a magnetic drive. Other types of computer-readable media may be used as well, such as those presently known in the art and those yet to be discovered. The method may comprise the following acts. Act 62 of the method may receive measured blood glucose (bG) levels of the person into a computing device, wherein the measured bG levels of the person are taken for two or more days such that at least one bG measurement is taken per day, and the at least one daily bG measurement corresponds to one or more daily events for the person. Act 64 of the method may record the measured bG levels in the computing device. Act 66 of the method may determine, by the computing device, whether the recorded bG levels are below, within, or above one or more predetermined bG ranges. And act 68 of the method may automatically provide, by the computing device, therapeutic guidelines to the person, based on whether the recorded bG levels are below, within, or above the one or more predetermined bG ranges. The acts of the method may be performed in any suitable order.

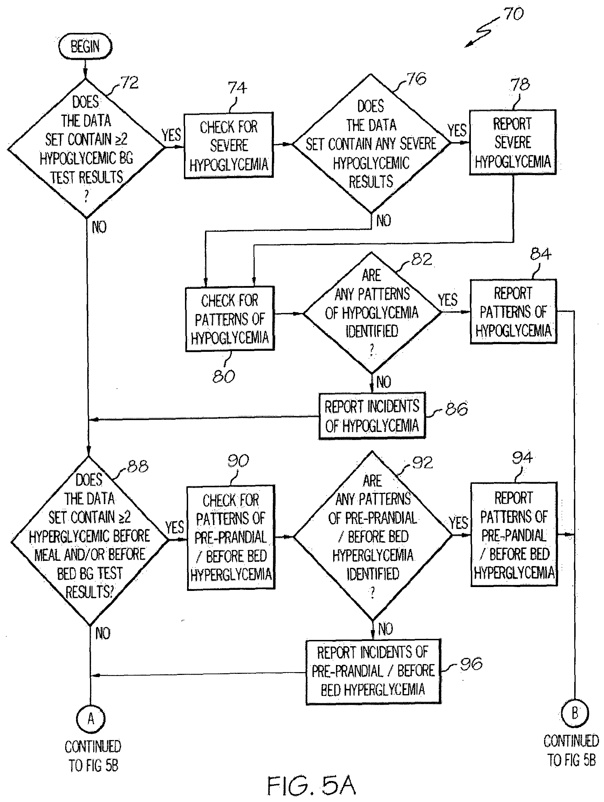

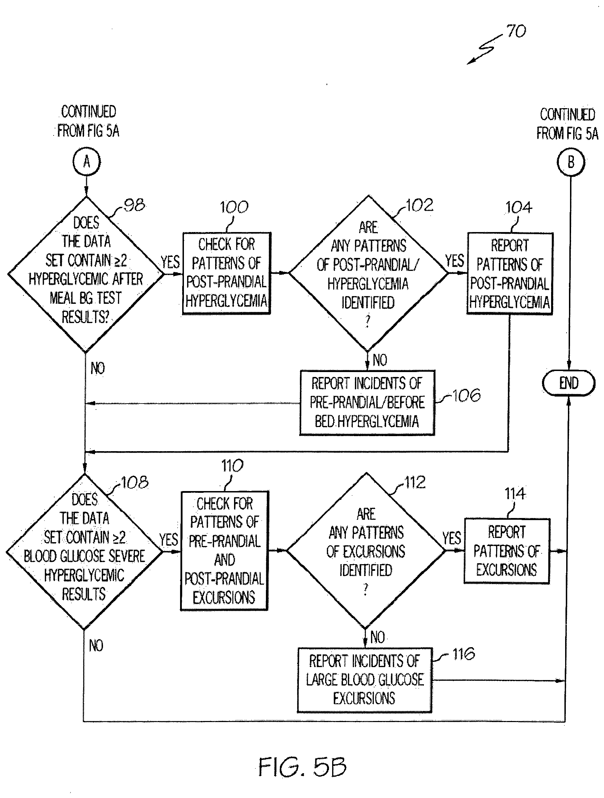

FIGS. 5A-B depict a flow diagram 70 of a method, according to one embodiment, for providing therapeutic guidelines based on whether the recorded bG levels are below, within, or above one or more predetermined bG ranges. As previously defined herein, a hypoglycemic bG level is one that is below the predetermined bG range, but by an amount that is less than the bG excursion amount; and a severe hypoglycemic bG level is one below the predetermined bG range by the bG excursion amount or more. Similarly, a hyperglycemic bG level is one that is above the predetermined bG range, but by an amount that is less than the bG excursion amount; and a severe hyperglycemic bG level is one that is above the predetermined bG range by the bG excursion amount or more.

Act 72 of the method determines whether two or more of the measured bG levels are considered hypoglycemic. If Yes, the flow diagram 70 advances to act 74; if No, the flow diagram 70 advances to act 88. At act 74, the bG results are checked for severe hypoglycemia. The flow diagram 70 then advances to act 76, where it is determined whether any bG levels are considered severe hypoglycemic. If Yes, then the severe hypoglycemic bG levels are reported to the operator at act 78. If No, the flow diagram 70 advances to act 80, wherein the bG levels are checked for patterns of hypoglycemia.

A "pattern" may occur, for example, if two or more hypoglycemic bG levels are found before or after the same daily event, such as after breakfast. As another example, a "pattern" may occur if two or more hyperglycemic bG levels are found before or after similar daily events, such as after meals (e.g., after breakfast, after lunch, and/or after dinner). Other definitions for "pattern" may be used as well.

Continuing with the flow diagram 70, act 82 of the method determines whether any hypoglycemic bG levels exhibit a pattern. If Yes, the flow diagram 70 advances to act 84, wherein the pattern (or patterns) of hypoglycemic bG levels is reported; the flow diagram 70 subsequently ends. If No, the flow diagram 70 advances to act 86, wherein the individual hypoglycemic bG levels are reported; the flow diagram 70 then advances to act 88.

At act 88, it is determined whether two or more of the bG measurement levels are considered hypoglycemic either at a pre-meal measurement or the pre- sleep (e.g., before bed) measurement. If Yes, the flow diagram 70 advances to act 90; if No, the flow diagram 70 advances to act 98. At act 90, the bG levels are checked for patterns of pre-meal or pre-sleep hyperglycemia. The flow diagram 70 then advances to act 92, wherein it is determined whether there are any patterns of pre-meal or pre-sleep hyperglycemia. If Yes, the flow diagram 70 advances to act 94, wherein the pattern (or patterns) of pre-meal or pre-sleep hypoglycemic bG levels is reported; the flow diagram 70 subsequently ends. If No, the flow diagram 70 advances to act 96, wherein the incidents of pre-meal and/or pre-sleep hypoglycemic bG levels are reported; the flow diagram 70 then advances to act 98.

Act 98 of the method determines whether two or more of the bG measurement levels are considered hyperglycemic at a post-meal (i.e., postprandial) bG level measurement. If Yes, the flow diagram 70 advances to act 100; if No, the flow diagram 70 advances to act 108. At act 100, the bG levels are checked for patterns of post-meal hyperglycemia. The flow diagram 70 then advances to act 102, wherein it is determined whether there are any patterns of post-meal hyperglycemia. If Yes, the flow diagram 70 advances to act 104, wherein the pattern of post-meal hyperglycemic bG levels is reported; the flow diagram subsequently advances to act 108. If No, the flow diagram 70 advances to act 106, wherein the individual post-meal hyperglycemic bG levels are reported; the flow diagram 70 then advances to act 108.

At act 108, it is determined whether two or more of the bG measurement levels are considered severe hyperglycemic. If Yes, the flow diagram 70 advances to act 1 10; if No, the flow diagram 70 ends. At act 110, the bG levels are checked for patterns of pre-meal or post-meal severe hyperglycemia. The flow diagram 70 then advances to act 1 12, wherein it is determined whether there are any patterns of pre-meal or post-meal severe hyperglycemia. If Yes, the flow diagram 70 advances to act 1 14, wherein the pattern (or patterns) of pre-meal or post meal severe hyperglycemic bG levels are reported; the flow diagram 70 subsequently ends. If No, the flow diagram advances 70 to act 116, wherein the individual pre-meal and/or post-meal severe hyperglycemic bG levels are reported; the flow diagram 70 then ends.

The acts of the flow diagram 70 may be performed in any suitable order. Furthermore, as described herein, any number of techniques may be employed to determine whether there is a "pattern" in the bG measurement levels. For example, if bG measurements are taken for three consecutive days, a pattern may be defined as two or more hypoglycemic or hyperglycemic bG measurements before or after the same event. In this example, two pre-breakfast hypoglycemic bG measurement levels constitute a pattern. Other ways of defining a pattern may be used as well. In another example having five consecutive days of bG measurements, a pattern may be defined as five anomalous (e.g., not within the predetermined bG range) bG measurements before or after the same event. In this example, five pre-sleep hyperglycemic bG measurement levels constitute a pattern. In yet another example having three consecutive days of bG measurements, a pattern may be defined as two or more anomalous bG measurements after any meal (e.g., breakfast, lunch, or dinner). In this example, one hyperglycemic bG measurement level after breakfast on the first day, and another hyperglycemic bG measurement level after lunch on the third day constitute a pattern. Thus, it is contemplated that a definition of a "pattern" is very broad and may encompass a number of factors.

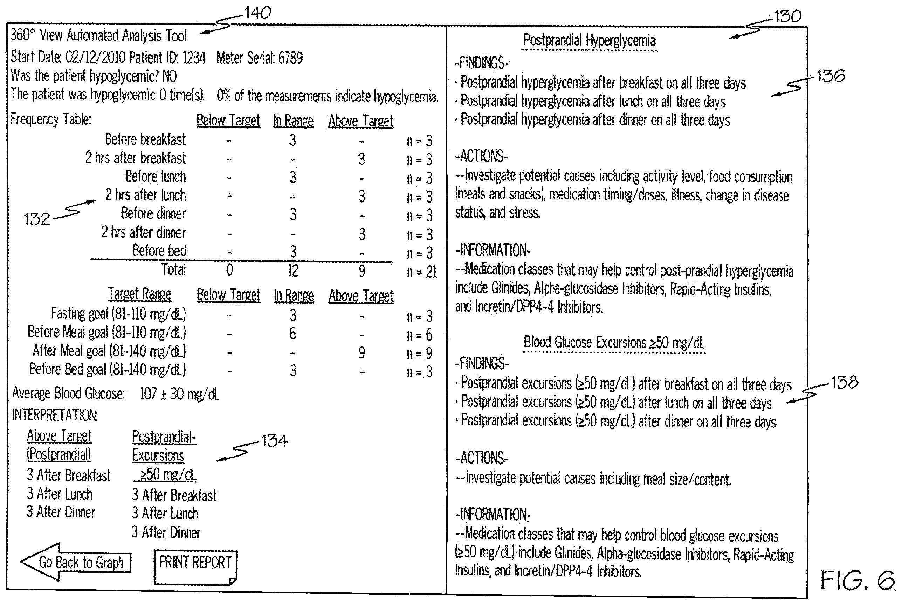

FIG. 6 depicts examples of therapeutic guidelines 130 according to one or more embodiments shown and described herein. The therapeutic guidelines 130 may be presented in graphic or textual form and may comprise a frequency table 132, a summary area 134, a hypoglycemic/hyperglycemic area 136, and a bG excursion area 138. The therapeutic guidelines 130 may further comprise an information area 140 which may provide basic information about the person and/or bG meter used.

The frequency table 132 may display the measured bG results in a tabular form and may be organized by the daily events to which each measurement corresponds. For example, the frequency table 132 may identify some or all of the following: (1) The number of hypoglycemic bG measurements for each time period, (2) The number of hyperglycemic bG measurements for each time period, (3) The number of normal bG measurements for each time period, (4) The total number of bG measurements for each time period, (5) The total number of hypoglycemic bG measurements, (6) The total number of hyperglycemic bG measurements, (7) The total number of normal bG measurements, and (8) The total number of bG measurements in the data set. As shown in FIG. 6, there may be a row in the frequency table 132 for before breakfast bG levels, after breakfast bG levels, etc. The bottom of the frequency table 132 may indicate the one or more predetermined bG ranges (called "Target Range" in the table). As an example, the pre-meal predetermined bG range may be approximately 81 mg/dl to approximately 110 mg/dl, and the post-meal and pre- sleep predetermined bG range may be approximately 81 mg/dl to approximately 140 mg/dl. Other predetermined bG ranges may be used as well.

The rows of the frequency table 132 may be labeled to indicate the time period (e.g., before breakfast, etc.) The columns of the time period frequency table may be labeled to indicate the range determination (e.g., below, within, or above the predetermined bG range). The summary frequency table may identify the number of hypoglycemic, normal, and hyperglycemic bG measurement levels for each of the following events: pre-breakfast, pre-lunch, pre-dinner, post-breakfast, post-lunch, post-dinner, and pre-sleeping. The rows of the summary frequency table may be labeled to indicate the time periods, while the columns may be labeled to indicate the range determination. Other ways of organizing the information may be used as well.

The summary area 134 may provide a synopsis of the recorded bG levels, as shown in FIG. 6. The hypoglycemic/hyperglycemic area 136 may indicate whether there were any hypoglycemic and/or hyperglycemic bG results. The hypoglycemic/hyperglycemic area 136 may provide text indicating findings, may propose actions, and may provide additional information when one or more hypoglycemic and/or hyperglycemic bG results are found. As an example, the hypoglycemic/hyperglycemic area 136 may suggest that the person, upon finding one or more hyperglycemic bG results, "Investigate potential causes including activity level, food consumption (meals and snacks), medication timing/doses, illness, change in disease status, and stress." The bG excursion area 138 may provide text indicating findings, may propose actions, and may provide additional information when one or more recorded bG levels are below or above the one or more predetermined bG ranges by at least the bG excursion amount (e.g., severe hypo- or hyperglycemic results). The bG excursion amount may be, for example, 50 mg/ml. In FIG. 6, as an example, the bG excursion area 138 may state, "Investigate potential causes including meal size/content. Other areas, both graphic and textual, may be included in the therapeutic guidelines 130.

The methods and systems described herein for providing therapeutic guidelines may permit the person having diabetes to modify some or all of the operating parameters on which the therapeutic guidelines may be based. Such operating parameters may include, but are not limited to, the starting and ending dates for the bG measurements, how many daily bG measurements are taken, which daily events correspond to the bG measurements, and so forth.

The methods and systems may also allow the person to enter relevant information about himself/herself and/or the bG meter, some of which may be displayed in the information area 140. As an example, the person may enter his/her name, the bG meter type, and the serial number of the bG meter, which may be stored in the memory along with the bG measurement levels. Furthermore, the person may enter information about how he/she is feeling, whether he/she is tired, etc. This latter type of information may be entered for each bG measurement, if desired, so that other patterns (other than those relating to the bG levels) may be recognized, either by the processor or by the person (e.g., upon seeing a report).

In addition to reporting incidents and patterns in the measured bG levels, the methods and systems described herein may also be configured to provide graphical information, either on a display or via a printer. For example, a graph of the person's pre-sleep bG level may be graphically shown for the two or more consecutive days (e.g., see the graph on the display 12 of FIG. 1). Alternatively, all measured bG levels may be graphically shown, such that each pre- and post-event bG levels have their own color or other identifying characteristic. As an example, all pre-breakfast bG levels may be depicted in red, all post-breakfast bG levels may be depicted in orange, etc. The graph may also highlight which bG measurements are normal, hypoglycemic, severe hypoglycemic, hyperglycemic, and/or sever hyperglycemic. The methods and systems described herein may provide therapeutic guidelines to the person, based on whether the recorded bG levels are below, within, or above the predetermined bG range. The therapeutic guidelines may also be based on whether the recorded bG levels are considered severe hypoglycemic or severe hyperglycemic (i.e., they are below or above the predetermined bG range by at least the bG excursion amount). The following examples illustrate how the therapeutic guidelines may be determined.

If the recorded bG levels contain two or more hypoglycemic levels and the recorded bG levels contains one or more severe hypoglycemic levels, the therapeutic guidelines may report the following finding: "SEVERE HYPOGLYCEMIA." If the recorded bG levels contain three hypoglycemic levels for the before breakfast time period, the guidelines may indicate a pattern of hypoglycemia and provide the following finding: "Preprandial hypoglycemia before breakfast on all three days." If the recorded bG levels contains exactly two hypoglycemic bG test results for the before breakfast time period, the guidelines may indicate a pattern of hypoglycemia and provide one of the following findings (depending on the days when the results occurred): "Preprandial hypoglycemia before breakfast on days 1 and 2," "Preprandial hypoglycemia before breakfast on days 2 and 3," or "Preprandial hypoglycemia before breakfast on days 1 and 3." The same may be done for bG results measured before lunch or dinner.

If the recorded bG levels contain three hypoglycemic bG test results for the after breakfast time period, the guidelines may indicate a pattern of hypoglycemia and provide the following finding: "Postprandial hypoglycemia after breakfast on all three days." If the recorded bG levels contains exactly two hypoglycemic bG test results for the after breakfast time period, the guidelines may indicate a pattern of hypoglycemia and provide one of the following findings (depending on the days when the results occurred): "Postprandial hypoglycemia before breakfast on days 1 and 2," "Postprandial hypoglycemia before breakfast on days 2 and 3," or "Postprandial hypoglycemia before breakfast on days 1 and 3." The same may be done for bG levels measured after lunch or dinner. If the recorded bG levels contain three hypoglycemic bG test results before the sleep time period, the guidelines may indicate a pattern of hypoglycemia and provide the following finding: "Hypoglycemia before sleep on all three days." If the recorded bG levels contains exactly two hypoglycemic bG test results for the pre- sleep time period, the guidelines may indicate a pattern of hypoglycemia and provide one of the following findings (depending on the days when the results occurred): "Hypoglycemia before sleep on days 1 and 2," "Hypoglycemia before sleep on days 2 and 3," or "Hypoglycemia before sleep on days 1 and 3."

If the recorded bG levels contain two or more hypoglycemic bG test results, but no pattern of hypoglycemia is identified, the guidelines may provide the incidents of hypoglycemia and provide the following finding: "There were two or more occurrences of hypoglycemia, but no pattern was detected." If the recorded bG levels contain two or more hypoglycemic bG test results and the recorded bG levels contain one or more severe hypoglycemic results, the guidelines may report the following guideline: "DETERMINE CAUSE IMMEDIATELY."

If any pattern of hypoglycemia is identified, the guidelines may suggest the following actions: "1) Investigate potential causes of hypoglycemia including activity level, food consumption (meals and snacks), medication timing/doses and illness. 2) Resolve prior to addressing other blood glucose abnormalities."

If the recorded bG levels contain two or more hypoglycemic bG test results, but no pattern of hypoglycemia is identified, the guidelines may suggest the following action: "Investigate potential causes of hypoglycemia including activity level, food consumption (meals and snacks), medication timing/doses and illness." If the recorded bG levels contains two or more hypoglycemic bG test results, the guidelines may provide the following information: "Medication classes that may cause hypoglycemia include: Sulfonylureas, Glinides, Long-Acting Insulins, Rapid- Acting Insulins, and various fixed dose insulin combinations." If any pattern of hypoglycemia is identified, the guidelines may report no findings, actions, or information for hyperglycemia or severe hyperglycemia. If no pattern of hypoglycemia is identified, and the recorded bG levels contain three hyperglycemic results for the before breakfast time period, the guidelines may indicate a pattern of preprandial / pre-sleep hyperglycemia and provide the following finding: "Preprandial hypoglycemia before breakfast on all three days." If no pattern of hypoglycemia is identified, and the recorded bG levels contains exactly two hyperglycemic bG test results for the before breakfast time period, the guidelines may indicate a pattern of hyperglycemia and provide one of the following findings (depending on the days when the results occurred):
"Preprandial hyperglycemia before breakfast on days 1 and 2," "Preprandial hyperglycemia before breakfast on days 2 and 3," or "Preprandial hyperglycemia before breakfast on days 1 and 3." The same may be done for bG levels measured before lunch or dinner.

If no pattern of hypoglycemia is identified, and the recorded bG levels contain three hyperglycemic bG test results for the before sleep time period, the guidelines may indicate a pattern of preprandial / pre-sleep hyperglycemia and provide the following finding: "Hyperglycemia before sleep on all three days." If no pattern of hypoglycemia is identified, and the recorded bG levels contains exactly two hyperglycemic bG test results for the before sleep time period, the guidelines may indicate a pattern of preprandial / pre-sleep hyperglycemia and provide one of the following findings (depending on the days when the results occurred):
"Hyperglycemia before sleep on days 1 and 2," "Hyperglycemia before sleep on days 2 and 3," or "Hyperglycemia before sleep on days 1 and 3."

If no pattern of hypoglycemia is identified, no pattern of preprandial / pre- sleep hyperglycemia is identified, and the recorded bG levels contain two or more before meal and/or before sleep hyperglycemic bG test results, the guidelines may provide incidents of preprandial / pre-sleep hyperglycemia and provide the following finding: "There were two or more occurrences of hyperglycemia, but no pattern was detected."

If no pattern of hypoglycemia is identified, and the recorded bG levels contain two or more before meal and/or before sleep hyperglycemic bG test results, the guidelines may suggest the following actions: " 1) Investigate potential causes of hyperglycemia including activity level, food consumption (meals and snacks), medication timing/doses, illness, change in disease status, and stress. 2) Resolve pre-meal and bedtime hyperglycemia before addressing postprandial hyperglycemia."

If no pattern of hypoglycemia is identified but the recorded bG levels contains two or more before meal and/or before sleep hyperglycemic bG levels, the guidelines may provide the following information: " 1) Medication classes that may help control fasting, preprandial, or pre-sleep hyperglycemia include:
Sulfonylureas, TZDs, Biguanides, Long- Acting Insulins, and various fixed dose insulin combinations. 2) Resolving pre-meal and bedtime hyperglycemia may reduce postprandial hyperglycemia." If any pattern of preprandial/before bed hyperglycemia is identified, the therapeutic guidelines may report not findings, actions, or information regarding postprandial hyperglycemia or severe hyperglycemia.

If no pattern of hypoglycemia is identified, no pattern of preprandial / pre- sleep hyperglycemia is identified, and the recorded bG levels contain three hyperglycemic bG test results for the after breakfast time period, the guidelines may indicate a pattern of post-hyperglycemia and provide the following finding:
"Postprandial hyperglycemia after breakfast on all three days." If no pattern of hypoglycemia is identified and no pattern of preprandial / pre-sleep hyperglycemia bG test results for the after breakfast time period is identified, the guidelines may indicate a pattern of postprandial hyperglycemia and provide one of the following findings (depending on the days when the results occurred): "Postprandial hyperglycemia after breakfast on days 1 and 2," "Postprandial hyperglycemia after breakfast on days 2 and 3," or "Postprandial hyperglycemia after breakfast on days 1 and 3." The same may be done for bG levels measured after lunch or dinner.

If no pattern of hypoglycemia is identified, no pattern of preprandial / pre- sleep hyperglycemia is identified, and the recorded bG levels contain two or more after meal hyperglycemic bG test results, the guidelines may provide incidents of postprandial hyperglycemia and provide the following finding: "There were two or more occurrences of hyperglycemia, but no pattern was detected."

If no pattern of hypoglycemia is identified, no pattern of preprandial / pre- sleep hyperglycemia is identified, and the recorded bG levels contains two or more after meal hyperglycemic bG test results, the guidelines may suggest the following action: "Investigate potential causes of hyperglycemia including activity level, food consumption (meals and snacks), medication timing/doses, illness, change in disease status, and stress."

If any pattern of postprandial hyperglycemia is identified, the guidelines may provide the following information: "Medication classes that may help control postprandial hyperglycemia include Glinides, Alpha-glucosidase Inhibitors, Rapid- Acting Insulins, and Incretin/DPP4-4 Inhibitors."

If no pattern of hypoglycemia is identified, no pattern of preprandial / pre- sleep hyperglycemia is identified, no pattern of postprandial hyperglycemia is identified, and the recorded bG levels contains two or more after meal hyperglycemic bG levels, the guidelines may provide the following information: "Medication classes that may help control postprandial hyperglycemia and blood glucose excursions〉 x mg/dL include Glinides, Alpha-glucosidase Inhibitors, Rapid- Acting Insulins, and incretin/DPP4-4 Inhibitors," where "x" is the bG excursion amount.

If no pattern of hypoglycemia is identified, no pattern of preprandial / pre- sleep hyperglycemia is identified, and the recorded bG levels contains three severe hyperglycemic bG levels (e.g., bG levels above the predetermined range by the bG excursion amount or more) from before breakfast to after breakfast, the guidelines may indicate a pattern of severe postprandial excursions and provide the following finding: "Postprandial excursions 〉 x mg/dL after breakfast on all three days," where "x" is the bG excursion amount. If no pattern of hypoglycemia is identified, no pattern of preprandial / pre-sleep hyperglycemia is identified, and the recorded bG levels contains exactly two severe hyperglycemic bG levels from before breakfast to after breakfast, the guidelines may indicate a pattern of large postprandial excursions and report one of the following findings (depending on the days when the excursions occurred): "Postprandial excursions 〉 x mg/dL after breakfast on days 1 and 2," "Postprandial excursions 〉 x mg/dL after lunch on days 2 and 3," or "Postprandial excursions 〉 x mg/dL after lunch on days 1 and 3," where "x" is the bG excursion amount. The same may be done for bG levels measured before and after lunch as well as before and after dinner.

If any pattern of postprandial severe hyperglycemic bG levels is identified, the guidelines may suggest the following action: "Please investigate potential causes of postprandial excursions 〉 x mg/dL including meal size/content" and/or "Medication classes that may help control postprandial hyperglycemia and blood glucose excursions 〉 x mg/dL include Glinides, Alpha-glucosidase Inhibitors, Rapid- Acting Insulins, and incretin/DPP4-4 Inhibitors," where "x" is the bG excursion amount.

If no pattern of hypoglycemia is identified, no pattern of preprandial / pre- sleep hyperglycemia is identified, no pattern of preprandial excursions is identified, no pattern of postprandial severe hyperglycemia is identified, and the recorded bG levels contains two or more blood glucose excursions 〉 x mg/dL, the guidelines may provide incidents of large blood glucose excursions and provide the following finding: "The patient has experienced blood glucose excursions 〉 x mg/dL at least two times, but no pattern was detected," where "x" is the bG excursion amount.

If no pattern of hypoglycemia is identified and no pattern of preprandial / pre-sleep hyperglycemia is identified, no pattern of preprandial severe hyperglycemia is identified, no pattern of postprandial hyperglycemia is identified, and the recorded bG levels include two or more severe hyperglycemic bG levels (e.g., blood glucose excursions 〉 x mg/dL, where "x" is the bG excursion amount), the guidelines may suggest the following actions: "1) Please investigate causes of postprandial excursions including meal size/content. 2) Please investigate potential causes of blood glucose excursions between meals including snacking, stress, illness, and medication compliance." Still yet another aspect concerns a method and system for quickly customizing structured testing protocols in an elegant and inexpensive manner. Most persons with Type 2 diabetes are medically managed by primary care physicians. Regardless of the therapeutic approaches used in primary care practices, the outcomes are generally sub-optimal. Medication adjustments made in primary care practices are usually made on the basis of hemoglobin Ale values. These values reflect an average of blood glucose values over time but do not give specific information on what the values actually were. In addition, the Ale level does not measure blood glucose variability which may contribute to the development of macrovascular complications. Unfortunately, need for therapy adjustment is not often recognized until Ale levels are significantly high, indicating a dramatic decline in glycemic control.

In contrast to the practices of primary care physicians, endocrinologists and diabetologists manage diabetes much more aggressively. In addition to tracking Ale levels, these experts may ask patients to perform a structured, self-monitoring blood glucose (SMBG) testing regimen. This allows the endocrinologist or other expert physician to determine why their patient's Ale values rise and what might be the most appropriate therapeutic approach to correct the problem. Structured testing provides substantial data, safely expedites treatment, and is cost effective. However, devising a structured testing protocol can be time-consuming for the physician, and the forms may not contain appropriately customized fields for the optimal use of the form.

Despite the advantages of structured testing, primary care practices do not commonly use the structured SMBG testing approach. This new approach to diabetes management is generally unfamiliar to primary care practices. Primary care physicians may not appreciate the potential benefits of structured testing and may perceive a lack of time as well as equipment to support structured testing.

Furthermore, primary care physicians may lack a familiarity with interpreting SMBG data. Primary care physicians cannot be expected to match the level of expertise of an endocrinologist or a diabetologist in the management of Type 2diabetes, but they are usually burdened with treating the majority of Type 2 diabetics. Thus, a need exists for additional support for structured SMBG testing to help guide primary care physicians and patients with Type 2 diabetes down the appropriate therapy paths, thereby avoiding costly clinical inertia and prolonged periods of suboptimal glycemic control.

In addition to improving therapy regimens, third party payers, such as health insurance companies, would like to promote increased adoption of structured testing. Medications typically used to address Type 2 diabetes are limited in scope of application and may not be appropriate in all situations. Unlike with structured testing data, hemoglobin Ale values alone make it difficult to identify which medications may be considered appropriate for treatment. To assess the efficacy of a particular medication using hemoglobin Ale data, a physician must typically wait three months to find out if the medication is working. Even if an appropriate medication is selected, a patient may or may not experience the desired response. For third party payers, current medication approval processes generally require prerequisite therapy steps for a patient before the patent can become a candidate for more costly options, such as more expensive medications. Utilizing a customized structured testing regimen can address a number of these issues by reducing the time required to judge the efficacy of particular medication protocols.

Patients also benefit from structured testing regimens. Patients with Type 2 diabetes treated by primary care physicians often receive little feedback on the impact of their lifestyle on the disease and often have an inadequate education about how lifestyle affects the disease. As will be appreciated, the system and method described below allows a patient to receive reliable and cost-effective feedback on their disease status in between visits with their physician, while learning throughout the process.

To address these and other issues, the system creates customized structured testing protocols based on blood glucose data provided by the user. With proper collection of the data, the system quickly identifies any abnormalities present in the given testing window and presents general therapeutic guidelines. The system suggests an appropriate therapeutic path for the patient, including contacting their physician if medication changes may be needed, and then provide a customized structured testing protocol to assess the efficacy of this path.

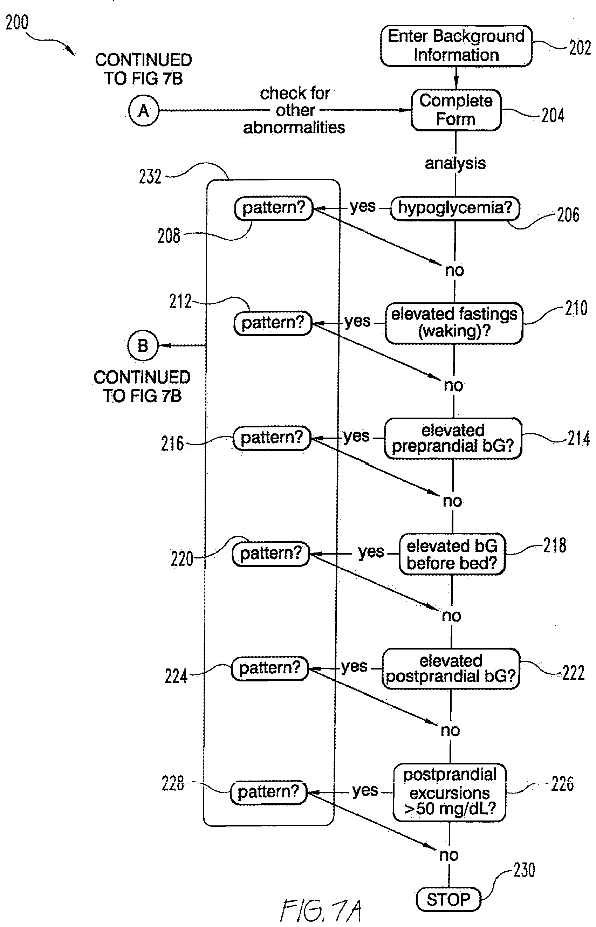

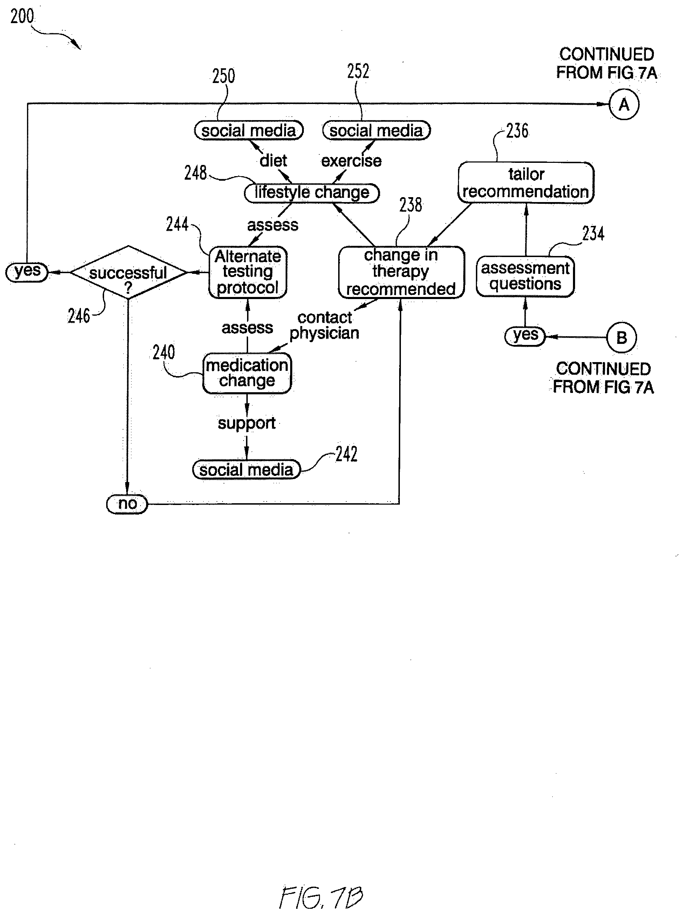

The technique for creating structured testing protocols will now be described with reference to a flowchart 200 shown in FIGS. 7 A and 7B. In one embodiment, this technique is generally performed in conjunction with the blood glucose meter 10 of the type such as illustrated in FIG. 1, the computing device 30 like the one illustrated in FIG. 2, or a combination of both, but it is envisioned that this technique can be performed with other types of devices. For the purpose of explaining this technique, most of the acts will be performed via a blood glucose meter 10 and computing device 30 owned by the patient or user, but again, these acts can be performed by other devices, such as a computing device 30 operated by the physician and/or a third party payer, to name just a few examples. In one particular example, a modified version of the ACCU-CHEK^{®} 360 View Blood Glucose Analysis system incorporating this unique technique will be used to address the problems identified. The computing device 30 includes a modified version of the ACCU-CHEK^{®} SmartPix and ACCU-CHEK^{®} 360 desktop software that, in addition to providing general statistical and data management abilities, provides an advice component for recommending changes to treatment regimens. However, it should be recognized that other hardware and/or software platforms can incorporate this technique. For instance, the meter 10 can be configured to perform all or some of the data entry and analysis. As another example, the software can reside on a hosted website so as to allow easier access to the data. It also can be appropriately integrated into a myriad of information management products. For example, this system can be used in conjunction with continuous glucose monitoring devices and/or insulin pumps that can be configured so as to be compatible with the overall system

Although the patient or physician will be described as entering in particular data into the computing device 30, it should be appreciated that other individuals, like physician assistants, relatives of the patient, and employees of the third party payer, can directly or indirectly enter the data into the computing device 30 or other systems. The structured testing forms described below in conjunction with this technique can be printed on paper using the printer of the computing device 30, but the forms can be provided in other manners and can come in other forms. For example, the forms or some modified version of the form can be shown on a display of the computing device 30 and/or the meter 10. As another example, the physician may also have a pre-printed collection of forms, and the physician or other health care provider simply pulls the appropriate form from their files and gives it to the user.

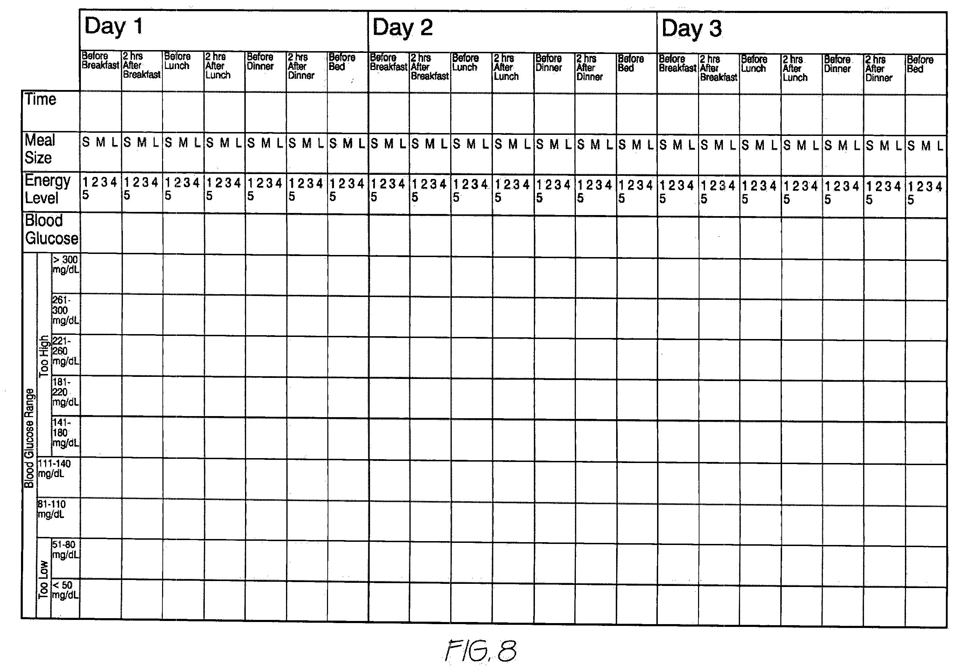

This customized structured testing protocol is developed utilizing information from various sources. As shown in FIG. 7A, to initiate the procedure, the user answers multiple demographic and lifestyle questions that are entered into the computing device 30 in stage 202. To name just a few examples, this background information can include information about medications being used, information related to comorbidity, and/or the duration the user has been a diabetic. The physician and/or patient also inputs various treatment goals and therapy parameters into the computing device 30. This information, which is stored in memory 34, is later used by the computing device 30 to determine the appropriate customized structured testing protocol. After entering the background information, the user in stage 204 completes an initial structured testing form. One example of an initial structured testing form is an ACCU-CHEK^{®} 360 View Blood Glucose Analysis System form, which is shown in FIG. 8, but it is contemplated that different forms can be used to provide an initial base line. In the example shown in FIG. 8, the user enters blood glucose readings from the meter 10 before and two hours after a meal along with the time when the reading was taken. The user also identifies the meal size, either small ("S"), medium ("M") or large ("L") as well as their energy level, with "1" signifying low, "2" signifying somewhat low, "3" signifying moderate, "4" signifying somewhat high, and "5" signifying high energy levels. The user charts the blood glucose readings in the "Blood Glucose Range" section of the form. In the illustrated example, the data is collected over a three day period, but in other examples, different time periods can be used. Moreover, other or different types of information can be collected with other types of structured testing forms. The user and/or physician enters the information from the structured testing form into the computing device 30 either manually or automatically. For instance, the user can type in the information into the computing device 30, or the computing device 30 can include a scanner that scans a completed paper version of the structured testing form. As another example, the blood glucose meter 10 automatically transfers the structured testing data to the computing device 30.

After the structured testing data is entered, the processor 32 of computing device 30 analyzes the data from the structured testing protocol for any adverse events as well as any patterns. Specifically, as shown in FIG. 7A, the computing device 30 in stage 206 determines whether hypoglycemia occurred. If so, the computing device in stage 208 determines whether there is a pattern for the hypoglycemic event. For example, the user may have a pattern of repeated, daily hypoglycemic events before breakfast (or other meals), but it should be recognized that other patterns may occur as well. As will be explained in greater detail below, if a pattern is detected, the computing device will then develop a customized recommended therapy regimen as well as structured testing protocol that will address the particular pattern at issue. If no hypoglycemia occurred in stage 206 and/or no pattern was detected in stage 208, the computing device 30 in stage 210 determines whether or not the user had any elevated blood glucose levels during fasting (waking) moments. If there were elevated levels, the computing device 30 determines whether there was a pattern in the readings in stage 212. For example, the data may show a pattern in which the user has repeated elevated blood glucose levels in the early morning. It is contemplated that the computing device 30 can detect other types of patterns in stage 212.

Assuming the computing device 30 does not detect elevated levels in stage 210 and/or a pattern of elevated levels in stage 212, the computing device 30 analyzes the data to determine whether there are elevated preprandial blood glucose levels (e.g., before dinner) in stage 214. If the user has elevated preprandial blood glucose levels, the computing device 30 then determines whether there is a pattern to the preprandial blood glucose levels. Otherwise, the computing device 30 in stage 218 analyzes the structured testing data to determine whether the user has elevated blood glucose levels before bedtime in stage 218. If so, the computing device 30 determines if there is a pattern to the elevated blood glucose levels before bedtime in stage 220. For example, the computing device 30 may notice a spike in blood glucose levels right before bedtime in all three days of the structured testing data used in conjunction with the form shown in FIG. 8.

When the blood glucose level is unelevated before bedtime in stage 218 or there is no pattern in stage 220, the computing device 30 in stage 222 analyzes the structured testing data to see if the user has elevated postprandial blood glucose levels, and if so, the computing device 30 in stage 224 determines whether there is a pattern. For instance, the user may have a pattern in which the blood glucose levels of the user are elevated after every lunch during the threeday structured test depicted by the form in FIG. 8. Of course, the computing device 30 can detect other types of patterns in stage 224. Assuming there is no elevated blood glucose levels in stage 222 nor a pattern in stage 224, the computing device 30 determines from the collected structured data in stage 226 memory 34 whether there were any postprandial excursions greater than a particular range. In the illustrated example, the range is 50 mg/dL, but in other variations, the range can be different based on the particular testing requirements. If there is a postprandial excursion in stage 226, the computing device 30 in stage determines whether there is a pattern to the excursions in stage 228. When there are no postprandial excursions in stage 226 nor patterns in stage 228, the computing device in stage 230 has determined that nothing needs to be addressed at this time. After stage 230, additional tests can be repeated in the manner as described above.

When a pattern is detected in any of the stages collectively identified by reference numeral 232, such as stage 208 or 228, the computing device 30 then proceeds to develop a customized change in therapy regimen as well as structured testing protocol. In one example, the measured blood glucose levels (or other collected data) are contextualized based on other collected information, and the pattern recognition is based on the contextualized data. To initiate the customization process, the computing device 30 via the display 36 or other output device asks the user questions for assessing a potential cause for the pattern in stage 234 (FIG. 7B). With the computing device 30 automatically detecting the pattern and automatically generating the customized assessment questions, the physician or other health care provider are free to devote their time to other activities. The computing device 30 asks the user a series of questions once an abnormality is identified to better guide the development of an appropriate therapeutic adjustment, ancillary support materials, and/or structured testing protocol for this abnormality. For example, the computing device may request additional information about particular medications taken, sleep habits, exercise, and/or insulin administration habits, to name just a few examples.

Based on the particular pattern 232 that initiated the customization process along with the assessment questions, the computing device 30 in stage 236 develops a tailored recommendation for a therapy regimen and/or testing protocol in stage 236. Instead of just basing the therapy recommendation purely on blood glucose levels, the recommendation in stage 236 is based on blood glucose level information in conjunction with other contextual data, such as the background information gather in stage 202, test data from stage 204, and the assessment questions in stage 234. In this context, different types of contextual data mean blood glucose measurements that are made under different circumstances. In particular, different circumstances can be defined as measurements made at different times of day (this is in contrast to "absolute or exact time" in which two non-simultaneous measurements are of course always different) and/or measurements timely related to different events, etc.

Different treatment therapies might be required depending on the relationships between the contextual data. By way of a nonlimiting example, a diabetic with a fasting blood glucose that is too high and a post lunch blood glucose level that is too low may require a different treatment therapy from a diabetic who has a fasting blood glucose that is too high as well as a post lunch blood glucose level that is too high. It should be appreciated that the pattern recognition in stage 232 and the tailored therapy recommendation in stage 236 can be based on one or more different types of contextual data as well as the relationship of values between the contextual data. For example, a pattern can be recognized in stage 232 and/or a tailored therapy recommendation can be created in stage 236 based on patterns related to the meal size, carbohydrates, time of day, activity level, energy level, and/or medication dosages in conjunction with the blood glucose levels. In stage 236, the particular pattern is automatically matched to a database of pre-diagnosed blood glucose patterns resulting in advice being provided to a health care professional, and this advice can be printed for a health care professional to give to the patient. For instance, when a scenario is identified which matches the current pattern or issue, the computer at a physician's office will produce a screen summarizing the diagnosis, cautions, evaluation of current medications effectiveness, suggestions of lifestyle, education and/or therapy interventions. The computer would then produce information for the health care professional to pass on to the patient containing motivational, lifestyle, diabetes condition, and/or medication related advice. When the computing device 30 determines that a change of medication is required, the computing device 30 alerts the physician and/or other health care providers of the recommended change to the medication in stage 240. For example, the computing device 30 can send an email to the physician alerting them of the recommended medication change. It should be appreciated that the physician can be alerted in other manners, such as receiving an alert on their computer screen, fax notice, a text, and/or a voicemail message, to name just a few examples. With the computing device 30 automatically generating a recommended change in therapy, the physician or other health care provide is able to more efficiently and effectively treat patients. Not only is one parameter considered during the development of the recommended therapy, such as fasting blood glucose levels, but other parameters are considered and various combinations of parameters are analyzed to lead towards more specific treatment protocols. For instance, a therapy recommendation can take into account a single parameter, such as fasting blood glucose levels, but also, the postprandial blood glucose levels as well as considering how the various parameters relate to one another. Consequently, this multidimensional assessment and recommendation technique facilitates enhanced personalized therapy. The physician in stage 240 can accept the proposed medication change or modify it based on the particular needs of the user. Once the change of medication is prescribed, social media 242 can provide support to the user and/or physician. For example, the social media 242 can include educational or other materials that are provided in an electronic and/or paper form.

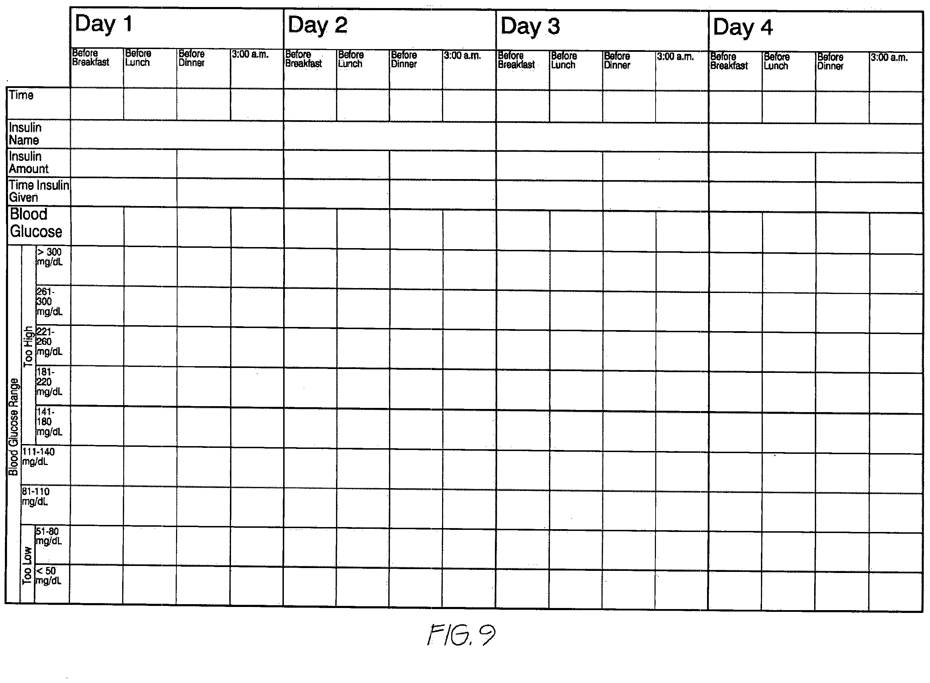

Along with the recommended change in medication, the computing device 30 in stage 244 provides an alternate structured testing protocol to assess whether or not the change in medication is effective. FIG. 9 illustrates an alternate structured testing form or protocol that is used to determine the effectiveness of intermediate- acting insulin. As can be seen, the form shares a number of features in common with the one depicted in FIG. 8, like collecting blood glucose levels. However, the form depicted in FIG. 9 collects different information, such as the insulin name, insulin amount, and the time the insulin was given. In addition, the testing time period is longer than in the form of FIG. 8; that is, four days instead of three days. However, it should be recognized that the computing device 30 can customize the structured testing protocol differently depending on the particular issue being addressed such that the form can collect different data. In stage 246, the user, physician, and/or other designee enters the information from the revised structured test (e.g., the form in FIG. 9) into the computing device 30, and the computing device 30 in stage 246 evaluates whether or not the change in treatment was successful in addressing the issue. In stage 246, the computing device 30 can evaluate for abnormalities in the fashion described above. For example, the computing device 30 can determine whether a hypoglycemia event (e.g., stage 206) occurred and/or there were some pattern (e.g., stage 232). Of course, the computing device 30 can evaluate for other issues in stage 246.

When the computing device 30 determines that the change of treatment was not successful, the computing device 30 in stage 238 recommends a different course of treatment. The computing device 30 can recommend a change of medication in stage 240, a lifestyle change in stage 248 or a combination of both. A recommended change of lifestyle in stage 248 is not generic, such as generally recommending more exercise; but instead, the recommendations are very specific, such as providing specific dietary and/or exercise regimens. For example, the computing device 30 in stage 248 can recommend that the user walk 10 miles a week and specify the recommended daily caloric intake. By providing specific guidelines, it is thought that the chance that the user will follow the guidelines will improve. Social media in stages 250 and 252 are respectively used to further support the user in achieving the recommended dietary and exercise goals.

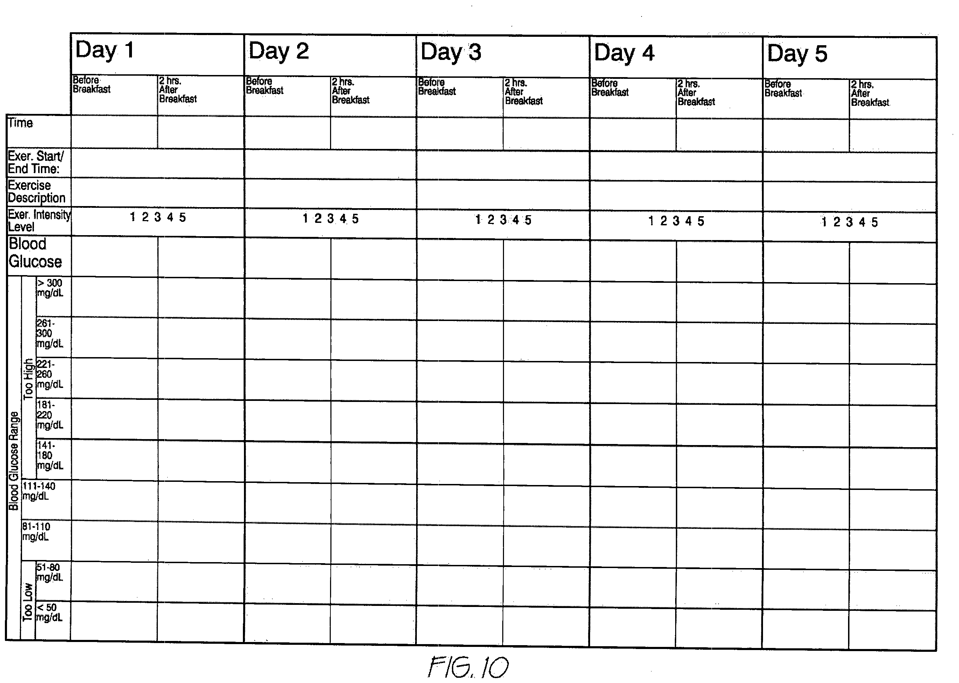

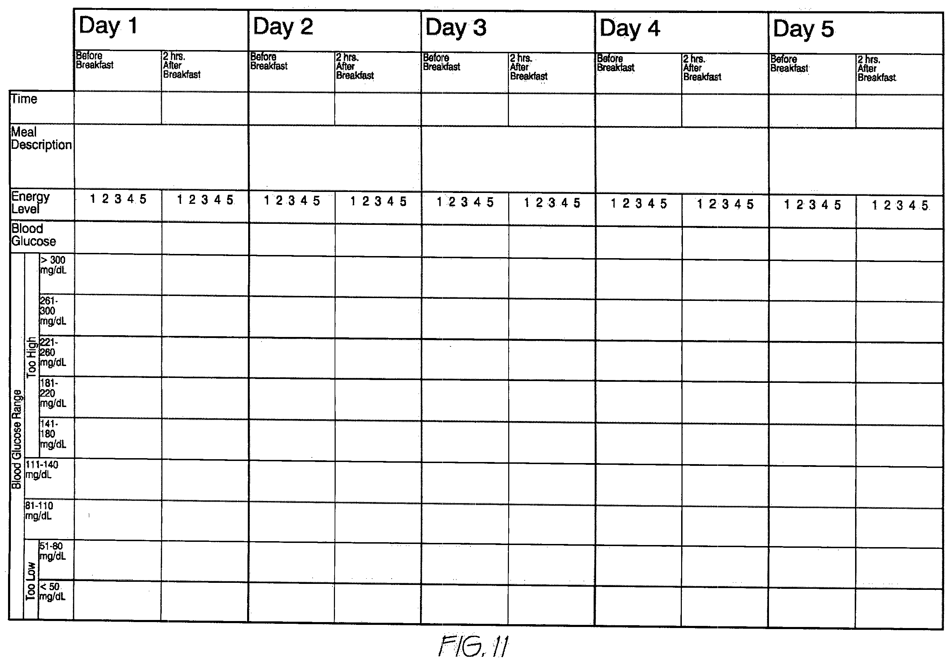

To determine the effectiveness of the recommended lifestyle change, the computing device 30 in stage 244 generates an alternate structured testing protocol depending on the abnormal pattern detected in stage 232 and the recommended lifestyle change from stage 248. By way of example, when the user has a pattern of hyperglycemia following breakfast, as is determined in stages 222 and 224, the computing device 30 recommends a structured testing regimen as represented by the forms in FIGS. 10 and 1 1, depending on the recommended lifestyle change in stage 248. FIG. 10 shows an example of a structured testing form that is used when the computing device 30 recommends an exercise change in stage 248. As shown, blood glucose levels are measured before and two hours after breakfast, and the corresponding measurement times are recorded. Via the form in FIG. 10, the user also provides information about the exercise length, intensity, and a description of the exercise. In this illustrated example, the structured testing occurs over five days, but a different number of days can be used in other examples. Moreover, other types of data can be collected in other examples. FIG. 11 shows an example of a structured testing form that is used when the computing device 30 recommends a dietary change in stage 248. The form in FIG. 11 is similar to the one depicted in FIG. 10 with the exception that the FIG. 11 form is used to record meal information and energy level rather than exercise-related information. Again, the data from these forms can be entered manually and/or automatically into the computing device 30. Based on the entered structured testing data, the computing device 30 in stage 246 determines whether the therapy change was successful in addressing the issue that caused the change in therapy. If not, depending on the results, the computing device proceeds to recommend another change of therapy in stage 238. When the computing device 30 in stage 246 determines that the recommended therapy modification successfully addressed the issue, the computing device proceeds to stage 204 so as to test for of abnormalities. The computing device 30 for the structured test in stage 204 can utilize the same standard form, such as the one depicted in FIG. 8, or a different one that has been modified based on the newly recommended treatment regimen. The computing device 30 proceeds in the same manner as described above to address any remaining treatment issues. Again, even when no patterns or other abnormalities are detected, a physician may periodically reinstitute the procedure to ensure that no new issues have arisen.

To help better show how this system and technique functions, several use case scenarios will be described below. These are just a few use case scenarios of the numerous use case scenarios that can occur.

In a first exemplary use case, a user, which for the purposes of discussion will be called "John", has a modified version of the ACCU-CHEK^{®} 360 desktop software on his personal computer (e.g., computing device 30) that communicates with a web hosted system that maintains patient records. John's computer asks a series of questions about his demographics, health status, disease status, medications, lifestyle, social media interests, and preferences (stage 202 in FIG. 7A). His physician also remotely inputs information on John's health goals and medications via the physician's office computer. Once the initial setup is complete, John is prompted by his home computer to complete a 360 View Blood Glucose analysis system form (stage 204). John prints the customized form from his home computer. The form is customized to reflect John's blood glucose target ranges, dates John will be performing the tests, his daily schedule, medications, doctor information, and various other items. After John completes the form, the data is entered into his computer. The data can be automatically downloaded from his blood glucose meter 10 and/or scanned from the manually filled out structured testing form into his computer. Once the data is downloaded to John's computer, the web hosted system can upload the data for processing. The system identifies fasting hyperglycemia as John's primary glucose abnormality (stage 210). A pattern of fasting hyperglycemia is identified and shared with John (stage 212). The computer asks John a few questions about medication and lifestyle choices related to this specific abnormality of fasting hyperglycemia and identified patterns (stage 234). A recommendation is made to the John to contact his physician regarding a potential medication adjustment (stages 236 and 238). John's physician modifies his therapy by adding LANTUS^{®} brand insulin (stage 240). John's physician inputs this change into the system via a web site. Based on data entered into the system, his computer asks John another series of questions related to the new testing protocol that has been recommended. The questions relate to upcoming events in John's life such as travel, illness, and other things that may impact the new structured test protocol the system is creating for John. The system via John's computer then generates a LANTUS^{®} titration form for John (stage 244), and he prints it from his home computer. The customized form reflects many things including the dates John will be performing the test, along with medication information, initial LANTUS^{®} dose as prescribed by John's physician, and John's personalized blood glucose target ranges. The system also makes social media recommendations for John (stage 242). During the setup, John indicated he enjoys reading and participating in internet blogs. The system provides links to various internet blogs related to other people initiating basal insulin. John continues the LANTUS^{®} titration protocol until his fasting blood glucose levels are within desired range. Data from the completed LANTUS^{®} titration forms are entered into the system. The system via his computer congratulates John on his completion of the LANTUS^{®} titration protocol and acknowledges that his fasting blood glucose levels are now within the goal range set by John and his physician. John is excited about his success and brags on the blog the system recommended to him for support. He receives lots of good feedback from fellow bloggers on the site. The system then recommends to John to complete an ACCU- CHEK ^{®} 360 View Blood Glucose Analysis System form (see e.g., FIG. 8) to check for any additional glucose abnormalities (stage 204).

All of the information entered into the web hosted system is stored for later use, so the system can identify habits, patterns, and abnormalities, and can recommend changes that have been successful for John in the" past.

In a second exemplary use case, a user, which for the purposes of discussion will be called "Jane", has a modified version of the ACCU-CHEK^{®} 360 desktop software on her personal computer (e.g., computing device 30) that communicates with a web hosted system that maintains patient records. The system via Jane's computer asks a series of questions about the Jane's demographics, health status, disease status, medications, lifestyle, social media interests, and preferences (stage 202). Jane's physician also inputs information on her health goals and medications via the physician's office computer: Once the setup is complete, Jane is prompted by the system to complete a 360 View Blood Glucose analysis system form (stage 204). Jane prints the customized form from her home computer. The form is customized to reflect Jane's blood glucose target ranges, dates Jane will be performing the tests, her daily schedule, medications, doctor information, and various other :items. After Jane completes the form, the data is entered into the system via the meter 10 and/or a scanner scans the form into the system. After the form is analyzed by the system, postprandial hyperglycemia is identified as Jane's primary glucose abnormality (stage 222). A pattern of postprandial hyperglycemia after breakfast is identified (stage 224). Via her computer, the system asks a few questions about Jane's medication and lifestyle habits (stage 234).

If postprandial hyperglycemia is severe, and the system calculates lifestyle choices are most likely the cause, not the primary cause, of hyperglycemia, a recommendation will be given to Jane to contact her physician (stage 238). If her physician makes a medication adjustment or addition (stage 240), a postprandial hyperglycemia medication monitoring form targeting breakfast will be generated for Jane (stage 244). The form will prompt Jane to check blood glucose levels before and after breakfast, and it also logs her medication dose and administration time (see e.g., FIG.9).

Otherwise, if postprandial hyperglycemia is not severe, the system targets lifestyle as the probable cause of Jane's glycemic abnormality. A recommendation is made to Jane to modify her lifestyle choices (stage 248). At this point, Jane can select exercise recommendations or dietary modifications. If dietary modifications are selected, specific dietary suggestions are given for breakfast meal options. Jane then has the option to print a "sample" breakfast menu. If exercise recommendations are selected, the system asks a few questions to assess Jane's mobility and lifestyle. Specific movement recommendations are given to be performed before breakfast. Jane has the option to print this "sample" exercise list. For this example, Jane selects dietary modification as her strategy to overcome her glycemic problem. The system creates a new testing protocol form for Jane based on her answers to various questions about her lifestyle, schedule, etc. (stage 244). This new form, which can be similar to the one shown in FIG. 11, instructs Jane to test her blood before and after breakfast and choose her breakfast foods from the list provided. The form also has a place for Jane to record her food consumption amount and which foods she chose to eat at breakfast The system makes social media recommendation (stage 250) for Jane also, based on her preferences, and provides links to various social medial areas of support with other users making dietary changes to control postprandial hyperglycemia. Regarding social media, Jane previously entered into the system that she enjoys being active in her community and attending discussion groups with her peers on topics that interest her. Therefore, the system refers Jane to some social group meetings in her area for people using dietary modifications to aide in their diabetes management. Once Jane completes the new structured testing protocol, she inputs her new data into system for analysis. The system analyzes the data (stage 246) and identifies that postprandial hyperglycemia after breakfast has been resolved. The system congratulates Jane. Jane is extremely excited and shares her success with the support group the system identified for her to attend. Jane is instructed by the system to complete another 360 View Blood Glucose Analysis System form to check for any additional glucose abnormalities (stage 204). All information that Jane has ever entered into the system is stored for later use, so the system can identify habits, patterns, and abnormalities, and can recommend changes that have been successful for her in the past. Jane prints the form from her home computer and cannot wait to get started. She hopes all of her glycemic abnormalities will be this easy to resolve.

In a third use case example, a person with Type 2 diabetes visits his general practitioner after completing a 3 day 21 point structured monitoring program. The patient is 62 years old and has a HbAlc level of 8.5 percent, a body mass index (BMI) of 35, and blood pressure of 140/90. The person is also taking Sulphonylurea. Based on the test, the computer at the physician's office graphically displays the blood glucose values, firstly highlighting a pattern of hypoglycemic events. The computer cautions the use of Sulphonylurea as it might be the cause of the hypoglycemic events. Instead, the system recommends to the physician prescribing Metformin, increase in exercise and dietary change in the mornings (stage 238). The system also highlights meal rises and lack of recovery, and it recommends prescribing a statin to decrease glucose absorption from food. The physician would then have printable advice for the patient about how their previous medication may have been causing them to feel unwell and even have hypoglycemic events during the night and that their new medication will not cause this issue so that the patient is not to be afraid to take the whole prescribed dose. The printable advice form can also indicate that the medication will reduce overall blood glucose values. The form also states that the patient's body is resistant to insulin which may lead to further medication in the future, but the best preventative measure is 30 minutes of exercise each day which would make the patient feel much better. The advice form further informs the patient that their body is naturally more insulin resistant in the morning, and they should try low carbohydrate meals and have a look at the differences in their tests two hours after breakfast.

As should be appreciated from the discussion above, this system and technique automatically creates customized forms, identifies blood glucose abnormalities, communicates with physicians, offers therapeutic changes, offers ancillary support materials, assimilates input information, and then customizes alternate structured testing protocols to assess the efficacy of the changes as part of physician patient control. This in turn helps to readily identify blood glucose abnormalities via structured SM BG data, solicit the patient and physician for further elaboration on external factors associated with the identified abnormality, recommend/accept new therapeutic paths, provide ancillary support materials and social media options for the user, and develops a customized structured testing protocol to assess this new therapeutic path. As noted before, the system will perform these functions infinitum until the user is in within all target ranges set up in the system.

This system and technique helps the physician by providing a decision support tool and by assisting in the identification of patient blood glucose abnormalities. The system also helps to simplify the job of the physician by outlining safe and appropriate treatment paths as well as by supporting non- pharmaceutical treatment paths with the patient without the need for physician intervention. The forms are also automatically customizable for specific patient blood glucose and other health parameter goals. Physician can make adjustments to medications and lifestyle factors, and send the changes directly to system without a patient appointment. Physicians can also review results of structured testing protocols and make further recommendations without patient appointments. The system helps to simplify medication selection by presenting medication classes that are appropriate for an identified abnormality. This system also allows for therapeutic choices to be based on actual blood glucose readings rather than on Ale values alone. This system also allows physicians to more efficiently use their time with patients and helps to avoid clinical inertia. Prior treatment authorizations are also expedited. The medical advice provided is more consistent, and health care professionals can educate themselves and their patients based on structured monitoring advice. Patients also benefit from this overall system. For instance, this system provides feedback that can prompt behavioral change. The patient assessment questions help to establish customized forms that eliminate unnecessary data entry by the user. The system also offers ancillary support materials to augment lifestyle habits. The customized structured testing form pinpoints primary glycemic abnormalities and works with the patient as well as the physician to identify lifestyle/medication changes to correct the abnormality. The system asks the patient to check carbohydrate content of meals, in view of a postprandial excursion. The system warns the patient of dangerous blood glucose levels and prompts them to call to their physician. The system can be used to educate the user by providing a list of possible events that may have exacerbated the high blood glucose levels. To address abnormalities through lifestyle modifications, the system can recommend a time of day where exercise could be appropriately used to manipulate high blood sugar levels as well as offer dietary suggestions for glycemic abnormalities. The form are customized to reflect the schedule and lifestyle of the user so testing is at intuitive times for user. This overall system can help to reduce the number and length of physician visits as well. Moreover, it can distinguish the ability of meters to produce and streamline collection of structured blood glucose testing data so that the patient uses the right meter for the job. The system can also be configured to determine software compatibility, and the customization aspects can be used to simplify the user interface.

Third party payers, such as health insurance companies, also benefit from the overall system. It helps to determine if medication choices are justified, and it also allows for quicker justification in favor or against certain medication coverage. Prerequisite medications can be targeted to an abnormality. Resources are efficiently used, and the number of physician visits is reduced. The system also fosters environment for personalized medicine by rapidly identifying and addressing issues. It also helps to provide education to the user which in turn may mitigate later drastic treatment options. For instance, the system can encourage frequent meter usage for those in the under-served Type 2 population. As a general matter, it is thought that this method of customizing structured testing data collection and treatment will provide superior results over HbAlc testing alone.

It should be recognized that the example use cases provided above are just a few examples in the universe of numerous other use cases. By way of non-limiting examples, other use cases can include: diabetic control issues with shift workers; travel issues; pregnancy issues; pediatric diabetes; illness issues; issues related to schedule changes; the start of school; new exercise programs; hyperglycemia before breakfast, lunch, and/or dinner; lifestyle modification in which diet causes hyperglycemia before breakfast, lunch, and/or dinner; lifestyle modification in which exercise causes hyperglycemia before breakfast, lunch, and/or dinner; medication modifications that cause hyperglycemia at bedtime; lifestyle modifications in which diet causes hyperglycemia at bedtime; lifestyle modifications in which exercise causes hypoglycemia before breakfast, lunch, and/or dinner; lifestyle modifications in which diet causes hypoglycemia before breakfast, lunch, and/or dinner; medication modifications that cause hypoglycemia at bedtime; lifestyle modifications in which diet causes hypoglycemia at bedtime; medication modifications in which rapid-acting insulin start and titration create issues; fast- acting insulin start and titration cause issues; long-acting insulin start and titration cause issues; mixed insulin start and titration cause issues; sulfonyurea start and titration creates control issues; and miscellaneous medicine issues.

The technique described above with reference to the flowchart 200 in FIGS. 7 A and 7B can be further modified so as to use the pattern detection in order to develop threat alerts. There are many situations when a patient undergoes a hypoglycemic event and does not know how the event was caused. There are also situations when a patient undergoes an undetected hypoglycemic event. In these instances, the patient does not feel well but does not test at the correct time to know they suffered from hypoglycemia. While continuous monitoring devices can be used to monitor blood glucose levels for hypoglycemic events, there are numerous instances where a hypoglycemic event may go undetected even with a continuous monitoring device. There are many aspects of using a continuous monitoring device that may cause it to not properly detect the hypoglycemic event. For instance, the continuous monitoring device has to be calibrated properly or the reading from the continuous monitoring device might be incorrect. Thus, there is not a clear guarantee that all of the blood glucose levels captured by the continuous monitoring device are accurate enough to detect hypoglycemic events.

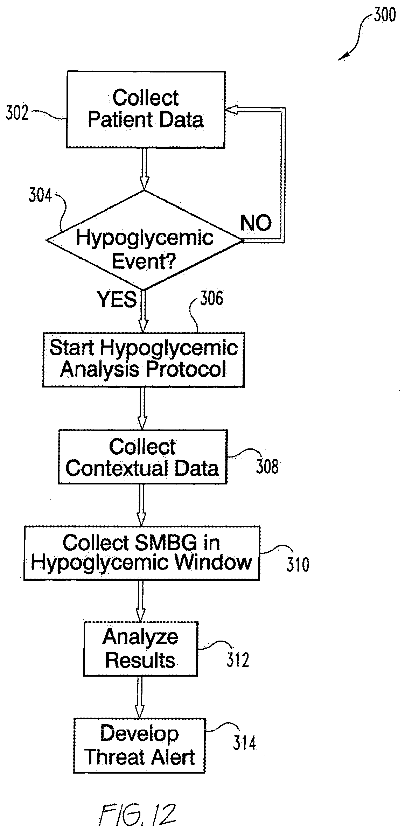

FIG. 12 shows a flowchart 300 that depicts a technique for developing a customized threat alert for a hypoglycemic event. This technique can be incorporated into the technique described above with reference to FIGS. 7 A and 7B. Moreover, it should be appreciated that this technique can be modified to detect conditions other than hypoglycemia. For the purposes of discussion, the technique will be described with reference to a system in which the glucose meter 10 is used to collect the blood glucose readings from the patient and the information is downloaded to the computing device 30, which in turn analyzes the data to develop a threat alert. However, other system configurations can be used. For example, in another variation, all of these acts can be performed using simply the glucose meter 10 by itself through which the contextual blood glucose information is entered and analyzed. In still yet another variation, the collected information can be uploaded and analyzed on a hosted website and/or server. A physician using a computing device 30 can then access the information on the website to determine whether the recommended threat alert is appropriate for the particular circumstance.

In stage 302, the patient collects blood glucose readings using the blood glucose meter 10. It should be recognized that these stages can be performed contemporaneously on a glucose meter or based on historical data when the glucose data is downloaded from the glucose meter 10 to the computing device 30. For example, the data from the glucose meter 10 can be downloaded to the computing device 30 using a modified version of the ACCU-CHEK^{®} 360 View software. In stage 304, the processor of the computing device 30 determines whether any of the downloaded data from the glucose meter 10 includes a hypoglycemic event. The hypoglycemic event can be detected through a single event and/or a pattern of hypoglycemic events (see e.g., stages 206 and 208 in FIG. 7A). If no hypoglycemic event is detected, the patient continues to collect data in the normal manner as in stage 302. However, if the computing device 30 detects a hypoglycemic event, the computing device 30 initiates a hypoglycemic analysis protocol in stage 306 in a fashion similar to the alternate testing protocol developed in stage 246 of FIG. 7B. The computing device 30 in one example programs or activates the glucose meter 10 to perform a structured testing protocol for detecting sources that may indicate the start of a hypoglycemic event. Based on the particular hypoglycemic pattern, the structured test typically (but not always) collects blood glucose and other related data in a window around when the hypoglycemic event normally occurs. In one particular example, the structured data testing protocol starts collecting data one hour before when the hypoglycemic is predicted or normally occurs and until three hours after the predicted hypoglycemic event. If for example the patient has a pattern of having hypoglycemia at 7:00 a.m., the patient would be instructed to conduct a structured test in which blood glucose levels are measured starting at 6:00 a.m. and ending at 10 a.m. In this particular example, the blood glucose measurements and other related information are entered at 15 to 20 minute intervals. It should be recognized that in other examples a different time ranges and/or different intervals can be used.

As discussed before, contextual data is used to identify the set of circumstances around which the blood glucose data is collected. The contextualized blood glucose information can then be used to determine what factors may lead to a hypoglycemic event. In stage 308, contextual data such as time of day, meal size, energy level, as well as other information that provides context or a set of circumstances around which the blood glucose measurements are collected within the window, is collected. In one example, this contextual data in stage 308 is collected using the computing device 30, but in other examples glucose meter 10 can be configured to collect this contextual information in addition to the glucose readings. Moreover, in other variations, a paper structured testing form can be used to collect the required information during the hypoglycemic analysis protocol.

Information from the paper form is then manually entered and/or automatically scanned into the computing device 30. Within the window, blood glucose level readings are also measured and recorded from the meter 10 in stage 310. The blood glucose and contextual information collected via the blood glucose meter 10 and/or the computing device 30 are analyzed with the computing device 30 in stage 312. The computing device 30 analyzes the test results to determine what pattern or factor instigates the hypoglycemic event. Specifically, the computing device 30 analyzes the result from the hypoglycemic analysis protocol to determine whether there are any particular patterns that would be a bellwether for a hypoglycemic event. For example, a patient routinely exercised before eating breakfast, which resulted in a pattern of hypoglycemic events before breakfast. While the computing device 30 may determine in some instances that a single factor is an indicator for a potential hypoglycemic event, the computing device 30 may determine that a combination of factors may cause a hypoglycemic event. For instance, the computing device 30 in stage 312 may determine that a particular meal in combination with a particular medication and glucose level is a strong indicator for a hypoglycemic event in a patient. Based on the analysis in stage 312, the computing device 30 can automatically download a threat alert protocol to the glucose meter 10 in stage 314 that automatically initiates an alert when a particular combination of factors indicative of a potential hypoglycemic event occur. When the glucose meter 10 analyzes the results in stage 312, the threat alert can automatically be initiated without the need of being downloaded from the computing device 30. It should be appreciated that the threat alert can be programmed into the meter 10, the computing device 30, and/or other systems in other manners.

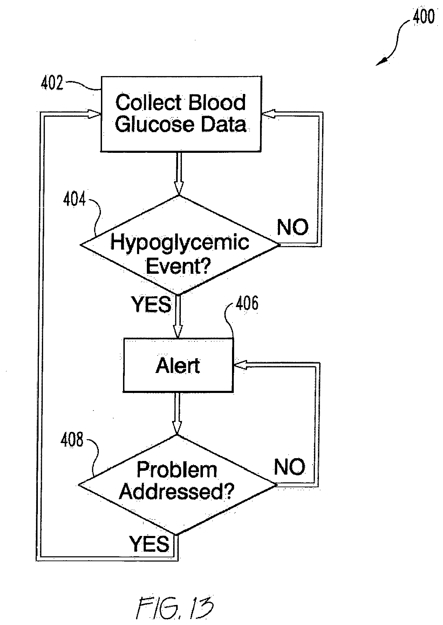

Flowchart 400 in FIG. 13 illustrates an example of a technique used for alerting a patient of a potential hypoglycemic event based on the threat alert developed using the technique illustrated with reference to flowchart 300 in FIG. 12. For explanation purposes, the technique will be performed using the glucose meter 10, but it is envisioned that other components, such as the computing device 30, can be used alone or in combination with the glucose meter 10 to perform this technique so as to alert the user. In stage 402, the blood glucose meter 10 collects blood glucose data and any other data, such as contextual data, needed to detect a hypoglycemic event. For example, if it was determined that a low caloric breakfast was the potential factor for causing a hypoglycemic event, the blood glucose meter 10 in addition to collecting blood glucose levels would request that the user enter the number of calories consumed during breakfast. Again, additional or alternative data may be collected as well, depending on the particular factors or indicators that would cause a hypoglycemic event. Based on the data collected, the glucose meter 10 in stage 404 determines whether a particular indicator for a threat of a hypoglycemic event was present. If no hypoglycemic threat indicator is detected, the glucose meter 10 proceeds to stage 402 to collect additional blood glucose data as would normally occur. Returning to our previous example, if for instance the user entered a breakfast caloric value that was above a hypoglycemic alert threshold, then the glucose meter 10 would collect blood glucose data in the usual fashion in stage 402. On the other hand, if a threat indicator is detected, such as the calories consumed at breakfast are too low, the blood glucose meter 10 can alert the patient of a potential hypoglycemic event in stage 406. It is also contemplated that others may be alerted to the potential event, such as the health care provider and/or other family member. As should be appreciated, hypoglycemic events are potentially life threatening and may result in unconsciousness. With another alerted about this potential hypoglycemic threat, the other person may be able to take preventive actions so as help the user avoid or remedy a hypoglycemic event. Along with the alert, the meter may instruct the user to perform certain acts, such as requesting they eat a specific meal and/or take a particular medication so as to avoid the onset of hypoglycemia. In stage 408, the glucose meter 10 determines whether the hypoglycemic problem has been addressed. For example, the patient or user may indicate that the patient followed the prescribed instructions and/or the blood glucose levels measured by the glucose meter indicate the hypoglycemic event has been averted. If the problem is not addressed, then the alert continues in stage 406, and if needed, the activity level may be increased to further address the issue. For example, if eating a particular meal did not address the hypoglycemic threat, the glucose meter 10 may instruct the patient to immediately seek medical attention. If the problem is addressed, the glucose meter proceeds to stage 402 to collect blood glucose data in the fashion as explained before.

This technique can be beneficial in a number of circumstances. By way of a non-limiting example, consider the possibility that the patient undergoes a hypoglycemic event immediately after breakfast. This technique can be used to analyze this particular situation. The patient would be encouraged to test his or her blood glucose values in a window around the hypoglycemic event (stages 308 and 310 in FIG. 12). This window would include a certain time before and a certain time after the hypoglycemic event. Typical examples would be one hour before and three hours afterwards respectively. The patient would be encouraged to take their blood glucose readings every 15 to 20 minutes in this window. Along with this information, the patient would also record their stress levels, the meal eaten, the amount of carbohydrates, the bolus given if any, any other medications, and the basal value if using an insulin pump. All of these values together would give an insight into the factors affecting the glycemia of the patient (stage 312). In this example, the detailed testing is carried out on three consecutive days. The rationale for this three day period being that the consecutive days would help average the behavior and also reveal whether there are differences in behavior across days. If this trend seems to persist, it would indicate an underlying problem. This would need correction either by a change in therapy or a change in lifestyle, as characterized by the contextual data. Using this information, the system can learn the pattern of hypoglycemia and potentially warn the patient the next time around the same time to take precautions against going hypoglycemic (see e.g., stage 406 in FIG. 13).

This technique can be used in conjunction with a continuous monitoring device that monitors blood glucose levels generally on a continuous basis.

Generally speaking, this technique can be used to determine whether the continuous monitoring device has detected a real or false hypoglycemic event. In either case, if the readings from the continuous monitoring device indicate hypoglycemia, the user can be retested through finger stick type measurements (e.g., via a discrete test glucose meter) to make sure that the hypoglycemic event was real. In another example, it is often the case where patients are put on a continuous monitoring device to establish their glycemic state. This is valid for both Type 1 and Type 2 diabetics. In some cases, it might be that the patient undergoes a hypoglycemic event while using the continuous monitoring device. However, there are many aspects to using a continuous monitoring device, one of which is that it has to be calibrated appropriately. Also, sometimes the reading might be different than those of the glucose meter so that it is not a clear guarantee that all behaviors captured by the continuous monitoring device are accurate. In this instance, a preliminary analysis of the continuous monitoring data can be carried out and then regions can be identified for further analysis. One of the key ones could be when the patient is hypoglycemic or experiences hypoglycemia-like symptoms (stage 304 in FIG. 12). In this case, the patient can be requested to test their blood glucose frequently in a window around this event (stage 310) and can also be requested to store their contextual data (stage 308). Using a combination of these two datasets, the cause of the hypoglycemia can be determined (stage 312). This combination analysis technique can be used as a starting point for discussion with the health care provider to see if there are changes that need to be made with regards to patient lifestyle and/or therapy.

It is noted that recitations herein of a component of the present invention being "configured" to embody a particular property or function in a particular manner, is a structural recitation, as opposed to a recitation of intended use. More specifically, the references herein to the manner in which a processor is "configured" denotes an existing physical condition of the processor and, as such, is to be taken as a definite recitation of the structural characteristics of the processor.

It is noted that terms like "preferably," "commonly," and "typically" are not utilized herein to limit the scope of the claimed invention or to imply that certain features are critical, essential, or even important to the structure or function of the claimed invention. Rather, these terms are merely intended to highlight alternative or additional features that may or may not be utilized in a particular embodiment of the present invention.

It should now be understood that the systems and methods described herein may provide therapeutic guidelines to a person having diabetes. While particular embodiments and aspects of the present invention have been illustrated and described herein, various other changes and modifications may be made without departing from the spirit and scope of the invention. Moreover, although various inventive aspects have been described herein, such aspects need not be utilized in combination. It is therefore intended that the appended claims cover all such changes and modifications that are within the scope of this invention.

## Claims

1. A method, comprising:
- detecting a pattern of an abnormality in blood glucose data collected from an individual with a computing device comprising a processor;
- generating a change in therapy recommendation for the individual with the computing device based on said detecting the pattern; and
- outputting a customized testing protocol customized to detect whether the change in therapy successfully addressed the abnormality with the computing device.

2. The method according to claim 1, further comprising receiving the blood glucose data with the computing device from a standardized structured testing data collection form before said detecting the pattern, preferably, further comprising:
- confirming the change in therapy successfully addressed the abnormality by analyzing data from the customized testing protocol with the computing device;
- instructing the individual to collect a second set of blood glucose data with the standardized structured testing data collection form with the computing device; and
- analyzing the second set of blood glucose data for a second abnormality pattern with the computing device.

3. The method according to any preceding claim, further comprising:
- receiving background information about the individual with the computing device; and
- wherein said generating the change in therapy recommendation includes selecting the change in therapy recommendation based at least in part on the background information.

4. The method according to any preceding claim, wherein said outputting the customized testing protocol includes providing advice related to the therapy recommendation.

5. The method of claim 1, further comprising:
- detecting the pattern for the blood glucose abnormality with the computing device based on the blood glucose data and contextual data collected from the individual, preferably, wherein the contextual data includes data about the individual surrounding collection of the blood glucose data.

6. The method of claim 1, wherein
- generating the change in therapy recommendation addresses the particular pattern at issue for the individual with the computing device based on said detecting the pattern and
- outputting the customized testing protocol is customized depending on the abnormality being addressed appropriate to detect whether the change in therapy successfully addressed the abnormality with the computing device; and
- the method further comprises determining, based on structured testing data entered according to the customized testing protocol, whether the change in therapy was successful in addressing the abnormality with the computing device.

7. A system, comprising:
- means for detecting a pattern of an abnormality in blood glucose data collected from an individual, wherein the means for detecting a pattern includes a computer device;
- means for generating a change in therapy recommendation for the individual based on the pattern; and
- means for outputting a customized testing protocol customized to detect whether the change in therapy successfully addressed the abnormality.

8. The system of claim 7, wherein
- the means for generating the change in therapy recommendation address the particular pattern at issue for the individual based on the pattern;
- the means for outputting the customized testing protocol are customized depending on the abnormality being addressed appropriate to detect whether the change in therapy successfully addressed the abnormality; and
- the system further comprises means for determining, based on structured testing data entered according to the customized testing protocol, whether the change in therapy was successful in addressing the abnormality.
